# EUROPÄISCHE PATENTSCHRIFT

(11) **EP 3 243 224 B1**
(45) Veröffentlichungstag und Bekanntmachung des Hinweises auf die Patenterteilung: **07.06.2023**
(21) Anmeldenummer: 15807806.3
(22) Anmeldetag: 08.12.2015
(51) Int. Cl.: H10K 10/50, H10K 19/00

(54) **ELEKTRONISCHES BAUELEMENT**
ELECTRONIC COMPONENT
COMPOSANT ÉLECTRONIQUE

(30) Priorität: 07.01.2015 DE 102015000120
(43) Veröffentlichungstag der Anmeldung: 15.11.2017
(73) Patentinhaber: Merck Patent GmbH, 64293 Darmstadt (DE)
(72) Erfinder: KIRSCH, Peer, 64342 Seeheim-Jugenheim (DE); TONG, Qiong, 64289 Darmstadt (DE); RUHL, Andreas, 64380 Rossdorf (DE); TORNOW, Marc, 81825 Muenchen (DE); BORA, Achyut, 10711 Berlin (DE)
(86) Internationale Anmeldenummer: PCT/EP2015/002477
(87) Internationale Veröffentlichungsnummer: WO 2016/110301

(56) Entgegenhaltungen:
- JP-A- 2008 021 685
- US-A1- 2005 099 209
- US-A1- 2014 008 601
- US-A1- 2014 027 698
- LAHANN J ET AL: "A reversible Switching Surface", SCIENCE, US AMERICAN ASSOCIATION FOR THE ADVANCEMENT OF SCIENCE, WASHINGTON, DC, Bd. 299, 17. Januar 2003 (2003-01-17), Seiten 371-374, XP002490005, DOI: 10.1126/SCIENCE.1078933
- FRANK SCHREIBER: "Structure and growth of self-assembling monolayers", PROGRESS IN SURFACE SCIENCE, OXFORD, GB, Bd. 65, Nr. 5-8, 1. November 2000 (2000-11-01), Seiten 151-256, XP009143276, ISSN: 0079-6816, DOI: 10.1016/S0079-6816(00)00024-1

## Beschreibung

Die Erfindung betrifft ein elektronisches Bauelement mit Schaltelementen, die eine molekulare Schicht mit einem vorzugsweise konformationsflexiblen molekularen Dipolmoment umfassen. Weitere Aspekte der Erfindung betreffen die Verwendung der molekularen Schicht sowie ein Verfahren zum Betrieb des elektronischen Bauelements.

In der Computertechnologie werden Speichermedien benötigt, die einen schnellen Schreib- und Lesezugriff auf darin abgelegte Informationen erlauben. Dabei erlauben solid-state Speicher bzw. Halbleiterspeicher die Realisierung besonders schneller und zuverlässiger Speichermedien, da keinerlei bewegliche Teile erforderlich sind. Derzeit werden hauptsächlich Dynamic Random Access Memory (DRAM) Speicher eingesetzt. DRAM erlaubt einen schnellen Zugriff auf die gespeicherten Informationen, jedoch müssen diese regelmäßig aufgefrischt werden, so dass beim Ausschalten der Stromversorgung die gespeicherten Informationen verloren gehen.

Im Stand der Technik sind auch nicht-flüchtige Halbleiterspeicher wie Flash-Speicher oder Magnetoresistive Random Access Memory (MRAM) bekannt, bei denen die Informationen auch nach dem Ausschalten der Stromversorgung erhalten bleiben. Dabei ist am Flash-Speicher nachteilig, dass ein Schreibzugriff vergleichsweise langsam erfolgt und die Speicherzellen des Flash-Speichers nicht beliebig oft gelöscht werden können. Typischerweise ist die Lebensdauer von Flash-Speicher auf maximal eine Million Schreib-Lese-Zyklen begrenzt. MRAM kann ähnlich wie DRAM verwendet werden und weist eine lange Lebensdauer auf, jedoch konnte sich dieser Speichertyp aufgrund der schwierigen Herstellungsverfahren nicht durchsetzen.

Eine weitere Alternative stellen Speicher dar, die auf Basis von Memristoren arbeiten. Der Begriff Memristor ist dabei aus den Englischen Wörtern "Memory" und "Resistor" (Speicher und Widerstand) zusammengesetzt und bezeichnet ein Bauelement, welches seinen elektrischen Widerstand reproduzierbar zwischen einem hohen und einem geringen elektrische Widerstand ändern kann. Dabei bleibt der jeweilige Zustand (hoher Widerstand oder geringer Widerstand) auch ohne eine Versorgungsspannung erhalten, so dass sich mit Memristoren nichtflüchtige Speicher realisieren lassen.

Eine wichtige alternative Anwendung elektrisch schaltbarer Bauelemente ergibt sich für das Gebiet des neuromorphen oder synaptischen Rechnens. In dort verfolgten Computer-Architekturen sollen die Informationen nicht klassisch sequentiell verarbeitet werden. Vielmehr wird angestrebt, die Schaltkreise hoch dreidimensional vernetzt aufzubauen, um eine Informationsverarbeitung analog zum Gehirn realisieren zu können. In derartigen, künstlichen neuronalen Netzwerken werden dann die biologischen Verbindungen zwischen Nervenzellen (Synapsen) durch die memristiven Schaltelemente dargestellt. Hierbei können unter Umständen auch zusätzliche Zwischenzustände (zwischen den digitalen Zuständen "1" und "0") von besonderem Nutzen sein.

Aus DE 11 2007 002 328 B4 ist ein elektrisch betätigbarer Schalter bekannt, der zwei Elektroden und eine aktive Region aufweist, die zwischen den beiden Elektroden angeordnet ist. Die aktive Region weist zwei primäre aktive Regionen auf, zwischen denen eine sekundäre aktive Region angeordnet ist. Die sekundäre aktive Region stellt eine Quelle bzw. eine Senke für ein ionisches Dotiermittel für die primären aktiven Regionen bereit. Durch Anlegen einer Spannung zwischen den beiden Elektroden können je nach Polarität Dotiermittel aus der sekundären aktiven Region in eine der primären aktiven Regionen injiziert werden. Je nach eingestellter Dotierung und Polarität weist die aktive Region eine hohe oder eine geringe elektrische Leitfähigkeit auf.

Nachteilig an dem bekannten, auf einer Leitfähigkeits- bzw. Widerstandsänderung basierenden elektrisch betätigbaren Schalter ist, dass das Bauelement nur für wenige Zyklen die gewünschte Funktionalität aufweist, jedoch nicht dauerhaft eine Memristor-Funktionalität bereitstellen kann.

Aufgabe war es daher weiterhin, nach neuen elektronischen Bauelementen zu suchen, die sich zur Anwendung in memristiven Vorrichtungen eignen und insbesondere im Hinblick auf eine oder mehrere der folgenden Eigenschaften Verbesserungen bringen:
- die Selektier- und Auslesbarkeit von zwei bezüglich ihres elektrischen Widerstands ausreichend unterschiedlichen Zuständen ("0", "1"), mit Hilfe eines elektrischen Feldes oder Stroms;
- die Schaltspannungen sollten im Bereich einiger 100 mV bis zu einigen V liegen;
- die Auslesespannungen sollten wesentlich kleiner als die Schreibspannungen (typisch 1/10 V) sein;
- der Lesestrom sollte mindestens 100 nA in Zustand "1" betragen;
- das Widerstandsverhältnis zwischen High-Resistance-State (HRS) (entspricht "0") und Low-Resistance-State (LRS) (entspricht "1"): R_{HRS}: R_{LRS} sollte mindestens größer gleich 10 sein, besonders bevorzugt sollte das Widerstandsverhältnis größer als 1000 sein.;
- die Zugriffszeiten für Schreib- und Lesevorgang sollten bei < 100 ns liegen;
- die Langzeitstabilität der selektierten Zustände bei Raumtemperatur ohne die Notwendigkeit, diese periodisch aufzufrischen und somit eine kontinuierliche Stromversorgung aufrecht zu erhalten, sollte auch bei kontinuierlicher Auslesung mehr als 10 Jahre betragen;
- ein breiter Temperaturbereich für Betrieb und Lagerung unter Erhalt der gespeicherten Information ist wünschenswert;
- ein hoher Grad an Reversibilität des Schaltvorgangs ohne "Ermüdungserscheinungen" (> 10⁶ Schaltzyklen) ist wünschenswert ("Endurance");
- das Potential zu hohen Integrationsdichten bis hinunter in den molekularen Größenmaßstab (< 10 nm) sollte gegeben sein;
- die Kompatibilität mit den Standardmethoden, -prozessen, Schaltungsparametern und -designregeln der Siliziumelektronik (CMOS) sollte möglich sein;
- eine einfache und damit kostengünstige Device-Architektur sollte möglich sein.

Es wurde nun gefunden, dass diese Aufgaben zumindest in Teilbereichen lösbar sind, wenn die Schaltelemente entsprechender Bauelemente eine molekulare Schicht aus dipolaren oder geladenen organischen Verbindungen enthalten.

In Angew. Chem. Int. Ed. 51 (2012), 4658 (H.J. Yoon et al.) und IACS 136 (2014) 16 - 19 (H.J. Yoon et al.) sind Anordnungen beschrieben, bei denen das elektronische Potential über Monoschichten von Alkylverbindungen mit polaren Endgruppen gemessen wird. Eine Eignung solcher Schichten zur Verwendung in Schaltelementen memristiver elektronischer Bauelemente lässt sich daraus nicht ableiten.

Weitere memristive Bauelemente werden in US2005099209, US2014008601, US2014027698 und JP2008021685 beschrieben.

Gegenstand der Erfindung ist daher ein elektronisches Bauelement, umfassend mehrere Schaltelemente, welche in dieser Reihenfolge
eine erste Elektrode,
eine molekulare Schicht, gebunden an ein Substrat, und
eine zweite Elektrode umfassen,
wobei die erste Elektrode das Substrat für die molekulare Schicht darstellt,
dadurch gekennzeichnet, daß die molekulare Schicht im Wesentlichen aus Molekülen (M) besteht, die eine Verbindungsgruppe (V) und eine Endgruppe (E) mit einer polaren oder ionischen Funktion enthalten und wobei die Moleküle (M) eine oder mehrere Verbindungen der Formel (I) darstellen,

   ~ (A)ₛ-(V)-[Y¹-(Z¹-Y²)ₘ]ₜ-(E) (I)

   worin
   - A: eine Ankergruppe;
   - V: eine C₁-C₂₅-Alkylengruppe, die in der Kette ein oder mehrere funktionelle Gruppen und/oder ein oder mehrere 3-bis 6-gliedrige, gesättigte oder teilweise ungesättigte, alicyclische oder heterocyclische Ringe enthalten kann und bei der ein oder mehrere H-Atome durch Halogen ersetzt sein können;
   - Y¹ und Y²: jeweils unabhängig voneinander eine aromatische oder eine alicyclische Gruppe mit vorzugsweise 4 bis 25 C-Atomen bedeuten, die auch kondensierte Ringe enthalten kann und die ein- oder mehrfach mit einer Gruppe R^{L} substituiert sein kann;
   - R^{L}: jeweils unabhängig OH, SH, SR°, -(CH₂)ₙ-OH, F, Cl, Br, I, -CN, -NO₂, -NCO, -NCS, -OCN, -SCN, -C(=O)N(R°)₂, -C(=O)R°, -N(R°)₂, -(CH₂)ₙ-N(R°)₂, gegebenenfalls substituiertes Silyl, gegebenenfalls substituiertes Aryl oder Cycloalkyl mit 6-20 C-Atomen oder lineares oder verzweigtes Alkyl, Alkoxy, Alkylcarbonyl, Alkoxycarbonyl, Alkylcarbonyloxy oder Alkoxycarbonyloxy mit 1-25 C-Atomen, in dem ein oder mehrere H-Atome durch F oder Cl ersetzt sein können und in dem zwei vicinale Gruppen R^{L} zusammen gegebenenfalls =O sein können;
   - n: 1, 2, 3 oder 4;
   - Z¹: jeweils unabhängig eine Einfachbindung, -O-, -S-, -CO-, -CO-O-, -O-CO-, -O-CO-O-, -OCH₂, -CH₂O-, -SCH₂-, -CH₂S-, -CF₂O-, -OCF₂-, -CF₂S-, -SCF₂-, -(CH₂)ₙ-, -CF₂CH₂-, -CH₂CF₂₋, -(CF₂)ₙ-, -CH=CH-, -CF=CF-, -C=C-, -CH=CH-COO-, -OCO-CH=CH- oder -CR°₂;
   - R°: jeweils unabhängig voneinander H oder C₁-C₁₂-Alkyl
   - m: 0, 1, 2, 3, 4, oder 5,
   - s und t: unabhängig voneinander 0 oder 1
bedeuten, und
∼die Bindung an das Substrat angibt,
und wobei die Endgruppe (E) eine polare Endgruppe ist, die mindestens eine Bindung aufweist, bei der die Elektronegativitätsdifferenz zwischen den beteiligten Atomen mindestens 0.5 beträgt, gewählt aus CN, SCN, NO₂, (C₁-C₄)-Haloalkyl, bevorzugt CF₃, (C₁-C₄)-Haloalkoxy, bevorzugt OCF₃, -S-(C₁-C₄)-Haloalkyl, bevorzugt SCF₃, S(O)₂-(C₁-C₄)-Haloalkyl, bevorzugt SO₂CF₃, SF₅, OSF₅, N(C₁-C₄-Haloalkyl)₂, bevorzugt N(CF₃)₂, N(CN)₂ und (C₆-C₁₂)-Haloaryl, bevorzugt Mono-, Di- oder Tri-fluorphenyl,
oder wobei die Endgruppe (E) eine redox-inaktive kationische oder anionische Gruppe ist, gewählt aus Imidazolium-, Pyridinium-, Pyrrolidinium-, Guanidinium-, Uronium-, Thiouronium-, Piperidinium-, Morpholinium-, Ammonium- und Phosphonium-Gruppen beziehungsweise Halogeniden, Boraten, Sulfonaten, Carboxylaten, Phosphaten, Phosphinaten, perfluorierten Alkyl-Sulfonaten und - Carboxylaten, Imidanionen und Amidanionen, bevorzugt gegebenenfalls substituierten Tetraphenylboraten, Tetrafluoroborat, Trifluoracetat, Triflat, Hexafluorophosphat, Phosphinaten und gegebenenfalls substituierten Tosylaten, wobei die Gegenionen der kationischen und anionischen Gruppen gewählt sind aus den aufgeführten anionischen beziehungsweise kationischen Gruppen.

Die Schaltelemente des elektronischen Bauelements sind insbesondere eingerichtet, zwischen einem Zustand mit hohem elektrischen Widerstand und einem Zustand mit geringem elektrischen Widerstand zu wechseln, wobei der Quotient zwischen hohem elektrischen Widerstand und niedrigem elektrischen Widerstand bevorzugt zwischen 10 und 100000 beträgt. Der elektrische Widerstand wird gemessen, indem eine Auslesespannung an das Schaltelement angelegt wird und der durch das Schaltelement fließende elektrische Strom gemessen wird. Der Wechsel zwischen den Zuständen erfolgt durch Anlegen einer Schaltspannung. Der Betrag der Auslesespannung ist geringer als der Betrag der Schaltspannung, wobei bevorzugt der Betrag der Auslesespannung maximal ein Zehntel des Betrags der kleinsten verwendeten Schaltspannung ist. Besonders bevorzugt ist es, wenn die Auslesespannung 10 bis 300 mV beträgt.

Weiterhin Gegenstand der Erfindung ist ein Verfahren zum Betrieb des erfindungsgemäßen elektronischen Bauelements, dadurch gekennzeichnet, dass ein Schaltelement des elektronischen Bauelements in einen Zustand hohen elektrischen Widerstands geschaltet wird, indem eine korrespondierende erste Elektrode auf ein erstes elektrisches Potential und eine korrespondierende zweite Elektrode auf ein zweites elektrisches Potential gelegt wird, wobei der Betrag der Spannung zwischen den beiden Elektroden größer ist, als eine erste Schaltspannung und das erste Potential größer als das zweite Potential ist, ein Schaltelement des elektronischen Bauelements in einen Zustand geringen elektrischen Widerstands geschaltet wird, indem eine korrespondierende erste Elektrode auf ein drittes elektrisches Potential und eine korrespondierende zweite Elektrode auf ein viertes elektrisches Potential gelegt wird, wobei der Betrag der Spannung zwischen den beiden Elektroden größer ist, als eine zweite Schaltspannung und das vierte Potential größer als das dritte Potential ist, und der Zustand eines Schaltelements bestimmt wird, indem zwischen korrespondierenden Elektroden eine Auslesespannung angelegt wird, deren Betrag kleiner als die erste und die zweite Schaltspannung ist und der fließende Strom gemessen wird.

Ebenso Gegenstand der Erfindung ist die Verwendung von erfindungsgemäßen Schaltelementen in einem memristiven elektronischen Bauelement.

Weiterhin Gegenstand der Erfindung ist die Verwendung von Molekülen der Formel (I) als molekulare Schicht in Schaltelementen eines memristiven elektronischen Bauelements.

### Kurze Beschreibung der Figuren.

Es zeigen
- Figur 1: eine erste Ausführungsform eines elektronischen Bauelements,
- Figur 2: eine zweite Ausführungsform eines elektronischen Bauelements,
- Figur 3: eine schematische Darstellung eines Versuchsaufbaus zur elektrischen Charakterisierung,
- Figur 4: ein Diagramm, welches eine zyklisch variierte Spannung darstellt.
- Figur 5: den aufgezeichneten Strom durch eine Probe mit dem Monolagensystem C6CN,
- Figur 6a: die zeitliche Abfolge der Widerstandswerte der Probe mit dem Monolagensystem C6CN,
- Figur 6b: das angelegte Spannungsprofil für den Zyklus der Figur 6a,
- Figur 7: den aufgezeichneten Strom durch eine Probe mit dem Schichtsystem Si-IL-1,
- Figur 8: den aufgezeichneten Strom durch eine Probe mit dem Schichtsystem Si-IL-3,
- Figur 9: den aufgezeichneten Strom für zwei Zyklen der I-U-Charakteristik einer Octyl-funktionalisierten ("C8") Si-Probe,
- Figur 10: den aufgezeichneten Strom durch eine Probe mit dem Schichtsystem Si-IL-3, mit permanenter zweiter Elektrode aus Pb/Ag.

Die erfindungsgemäßen Schaltelemente eignen sich zur Verwendung in elektronischen Bauelementen, insbesondere memristiven Bauelementen, welche die oben aufgeführten vorteilhaften Eigenschaften zeigen.

### Molekulare Schicht

Erfindungsgemäß in dem elektronischen Bauelement eingesetzte Schaltelemente umfassen eine molekulare Schicht, enthaltend Moleküle (M) wobei die Moleküle (M) eine oder mehrere Verbindungen der Formel (I) darstellen und wobei die Moleküle vorzugsweise unterschiedliche Konformationen einnehmen können und ein konformationsabhängiges molekulares Dipolmoment aufweisen.

Die erfindungsgemäß eingesetzte molekulare Schicht ist bevorzugt eine molekulare Monoschicht.

In einer Ausführungsform handelt es sich dabei um eine selbstorganisierte Monoschicht (SAM, self-assembled-monolayer).

In einer weiteren Ausführungsform wird die molekulare Schicht durch Chemisorption, insbesondere durch eine Additionsreaktion oder Kondensationsreaktion an das Substrat gebunden.

In einer weiteren Ausführungsform ist die molekulare Schicht durch Physisorption an das Substrat gebunden.

In einer weiteren Ausführungsform ist die molekulare Schicht mit 1 bis 10, bevorzugt 1 bis 5, besonders bevorzugt 1 bis 3 weiteren Lagen an organischen oder anorganischen Adsorbaten bedeckt. Geeignet sind beispielsweise Schichten aus Dielektrika, zum Beispiel oxidische oder fluoridische Materialien wie TiO₂, Al₂O₃, HfO₂, SiO₂ und LiF, oder Metallen wie Au, Ag, Cu, Al und Mg. Solche Schichten können durch definierte und atomgenaue Abscheidung, beispielsweise durch ALD (atomic layer deposition) Verfahren, in einer Dicke von wenigen Nanometern aufgebaut werden.

Die Verbindungsgruppe (V) ist bevorzugt konformativ flexibel, wodurch die Moleküle unterschiedliche Konformationen einnehmen können und ein konformationsabhängiges molekulares Dipolmoment aufweisen."Konformativ flexibel" bedeutet, dass die Verbindungsgruppe (V) so gewählt ist, dass sie mindestens zwei unterschiedliche Konformationen einnehmen kann.

In einer besonders bevorzugten Ausführungsform ist die Verbindungsgruppe (V) eine lineare oder verzweigte C₁-C₂₅-Alkylengruppe, in der eine oder mehrere nicht benachbarte CH₂-Gruppen jeweils durch -C=C-, -CH=CH-, -NR'-, -O-, -S-, -CO-, -CO-O-, -O-CO-, -O-CO-O- oder einen 3-6 gliedrigen, gesättigten oder teilweise ungesättigten, alicyclischen oder heterocyclischen Ring ersetzt sein können, wobei N, O und/oder S nicht direkt aneinander gebunden sind, in der ein oder mehrere tertiäre Kohlenstoffatome (CH-Gruppen) durch N ersetzt sein können und in der ein oder mehrere Wasserstoffatome durch Halogen ersetzt sein können, wobei R' jeweils unabhängig voneinander H oder C₁-C₁₂-Alkyl bedeutet.

Ganz besonders bevorzugt ist die Verbindungsgruppe (V) eine lineare oder verzweigte C₁-C₁₀-Alkylengruppe, in der eine oder mehrere nicht benachbarte CH₂-Gruppen jeweils durch -O-, -S-, oder einen 3-6 gliedrigen, gesättigten alicyclischen Ring ersetzt sein können, wobei O und/oder S nicht direkt aneinander gebunden sind, und in der ein oder mehrere Wasserstoffatome durch F und/oder Cl ersetzt sein können.

Die polare oder ionische Endgruppe ist so gewählt, dass sie dem Molekül ein permanentes Dipolmoment von mindestens 0,5 Debye verleiht. Bevorzugt ist das permanente Dipolmoment größer als 2 Debye und besonders bevorzugt größer als 3 Debye.

Die Elektronegativitätswerte werden nach Pauling bestimmt.

In einer Ausführungsform ist die Endgruppe (E) eine ionische Gruppe, d.h. eine kationische oder anionische Gruppe. Die ionische Gruppe ist redox-inaktiv, wobei redox-inaktiv erfindungsgemäß bedeutet, dass sie, in Kombination mit dem Gegenion, ein elektrochemisches Fenster von mindestens 2,0 V, bevorzugt mindestens 3,0 V, besonders bevorzugt von mindestens 4,0 V aufweist. Das elektrochemische Fenster gibt dabei die Stabilität gegen elektrochemische Reduktions- und Oxidationsprozesse an.

Besonders bevorzugt als ionische Gruppen sind: 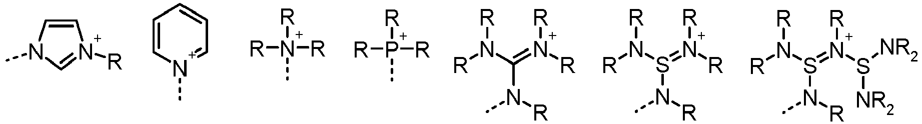 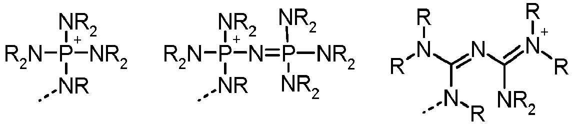 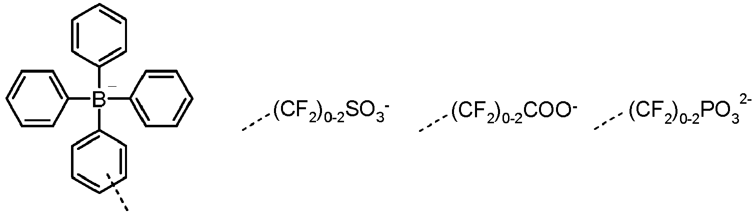 wobei
R C₁-C₁₂-Alkyl, C₆-C₁₄-Aryl, gegebenenfalls substituiert durch Halogen, C₁-C₄-Alkyl und/oder C₁-C₄-Oxaalkyl, bedeutet, wobei in Aromaten CH durch N ersetzt sein kann.

In einer bevorzugten Ausführungsform umfassen die Moleküle der Formel (I) eine Ankergruppe (A), über welche die Verbindungsgruppe an das Substrat gebunden ist.

In einer bevorzugten Ausführungsform ist die Ankergruppe (A) über eine kovalente Bindung an das Substrat gebunden.

In einer weiteren Ausführungsform ist die Ankergruppe (A) über Physisorption an das Substrat gebunden.

Bevorzugt ist die Ankergruppe (A) gewählt aus Carboxylat-, Phosphonat-, Alkoholat-, Arylat-, bevorzugt Phenolat-, Thiolat- und Sulfonatgruppen oder Fullerenderivaten, bevorzugt [60]PCBM ([6,6]-Phenyl-C61-butansäure-methylester) und [70]PCBM ([6,6]-Phenyl-C71-butansäuremethylester).

Die Herstellung der molekularen Schicht erfolgt beispielsweise durch Physisorption nach bekannten Methoden über van der Waals-Wechselwirkung oder elektrostatisch, beispielsweise durch Adsorption molekularer Anionen auf eine Poly-L-Lysin-Schicht, d.h. ein Polykation, wie sie beispielsweise für die Physisorption von DNA eingesetzt wird.

Bevorzugt sind die Moleküle der molekularen Schicht kovalent an das Substrat gebunden. Die Anbindung erfolgt nach bekannten, dem Fachmann geläufigen Methoden, beispielsweise durch Addition eines geeigneten Precursors an das Substrat oder durch Kondensation eines Precursors, der eine Gruppe A - LG aufweist, wobei A eine erfindungsgemäße Ankergruppe ist und LG eine geeignete Abgangsgruppe darstellt.

Für Additionsreaktionen kann beispielsweise eine geeignetes Substrat, bevorzugt eine Siliziumoberfläche - nach entsprechender Vorbehandlung mit wässriger NH₄F-Lösung - behandelt werden, um eine wasserstoffterminierte Oberfläche zu erhalten. Die so behandelte Oberfläche kann dann bei erhöhter Temperatur unter Sauerstoffausschluss entweder direkt mit einem geeigneten flüssigen Precursor oder einer Lösung des Precursors in einem geeigneten Lösungsmittel behandelt werden. Geeignete Precursoren sind in diesem Fall Verbindungen mit einer terminalen C-C-Doppelbindung.

Geeignete Precursor-Verbindungen sind teilweise kommerziell erhältlich oder können nach bekannten, dem Fachmann geläufigen Methoden, wie sie beispielsweise in Houben Weyl, Methoden der organischen Chemie, Georg Thieme Verlag, Stuttgart 2004, beschrieben sind, synthetisiert werden.

Geeignet als Precursoren sind beispielsweise die folgenden Verbindungen, die das Prinzip der Precursorwahl veranschaulichen sollen:

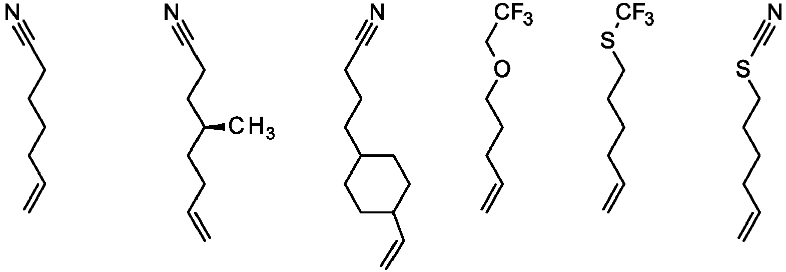

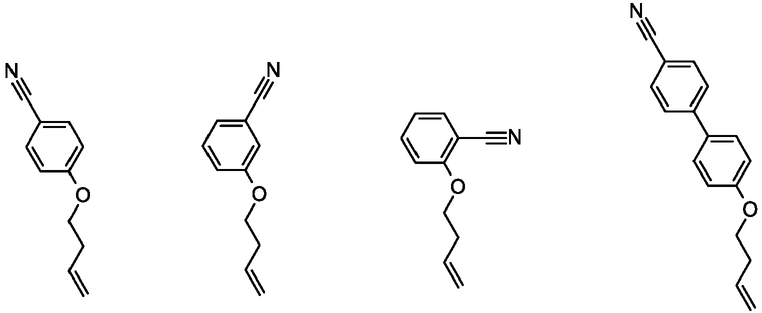

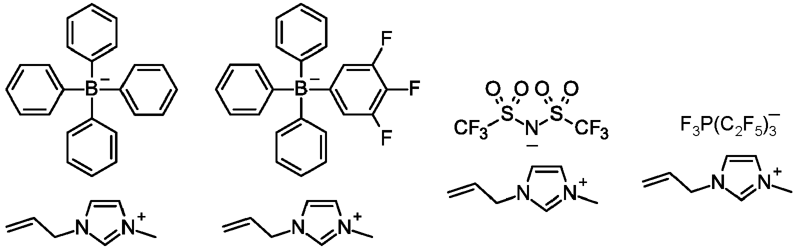

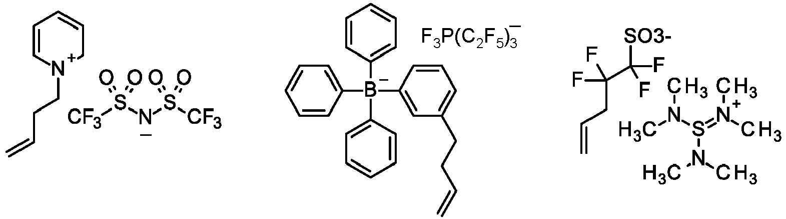

### Substrat

In den erfindungsgemäßen Schaltelementen sind die Moleküle der molekularen Schicht an ein Substrat oder eine zwischen molekularer Monoschicht und Substrat befindliche Zwischenschicht gebunden. Das erfindungsgemäße Substrat kann je nach Aufbau der Schaltelemente unterschiedliche Funktionen wahrnehmen. Beispielsweise kann ein leitfähiges Substrat als erste Elektrode dienen. Ebenso kann das Substrat eine Schicht einer Diode darstellen.

Als Substrat eignen sich beispielsweise
Elementhalbleiter wie Si, Ge, C (Diamant, Graphit, Graphen, Fulleren), α-Sn, B, Se und Te und/oder
Verbindungshalbleiter, bevorzugt Gruppen III-V-Halbleiter wie GaAs, GaP, InP, InSb, InAs, GaSb, GaN, AlN, InN, AlₓGa₁₋ₓAs und InₓGa₁₋ₓNi, Gruppen II-VI-Halbleiter wie ZnO, ZnS, ZnSe, ZnTe, CdS, CdSe, CdTe, Hg₍₁₋ₓ₎Cd₍ₓ₎Te, BeSe, BeTeₓ und HgS;
Gruppen III-VI-Halbleiter wie GaS, GaSe, GaTe, InS, InSeₓ und InTe, Gruppen I-III-VI-Halbleiter wie CuInSe₂, CuInGaSe₂, CuInS₂ und Culn-GaS₂;
Gruppen IV - IV-Halbleiter wie SiC undSiGe und
Gruppen IV-VI-Halbleiter wie SeTe,
organische Halbleiter wie Polythiophen, Tetracen, Pentacen, Phthalocyanin, PTCDA, MePTCDI, Chinacridon, Acridon, Indanthron, Flaranthron, Perinon, AlQ₃- und Mischsysteme wie PEDOT:PSS und Polyvinylcarbazol/TLNQ-Komplexe,
Metalle, wie Au, Ag, Cu, Al und Mg,
leitfähige oxidische Materialien wie Indiumzinnoxid (ITO), Indiumgalliumoxid (IGO), InGa-α-ZnO (IGZO), aluminiumdotiertes Zinkoxid und zinndotiertes Zinkoxid (TZO), fluordotiertes Zinnoxid (FTO) und Antimonzinnoxid.

Die molekulare Schicht kann optional auch an eine dünne (bevorzugt 0.5 - 5 nm dick) oxidische oder fluoridische Zwischenschicht, z.B. TiO₂, Al₂O₃, HfO₂, SiO₂ oder LiF gebunden sein, die sich auf dem Substrat befindet.

Die Gegenelektrode bzw. zweite Elektrode besteht aus einem leitenden oder halbleitenden Material oder einer Kombination (Schichtstapel) mehrerer dieser Materialien. Beispiele sind die als Substratmaterial aufgeführten Materialien. Bevorzugt sind Hg, In, Ga, InGa, Ag, Au, Cr, Pt, PdAu, Pb, Al, Mg, CNT, Graphen und leitfähige Polymere (wie PEDOT:PSS).

In der nachfolgenden Beschreibung der Ausführungsbeispiele der Erfindung werden gleiche oder ähnliche Komponenten und Elemente mit gleichen oder ähnlichen Bezugszeichen bezeichnet, wobei auf eine wiederholte Beschreibung dieser Komponenten oder Elemente in Einzelfällen verzichtet wird. Die Figuren stellen den Gegenstand der Erfindung nur schematisch dar.

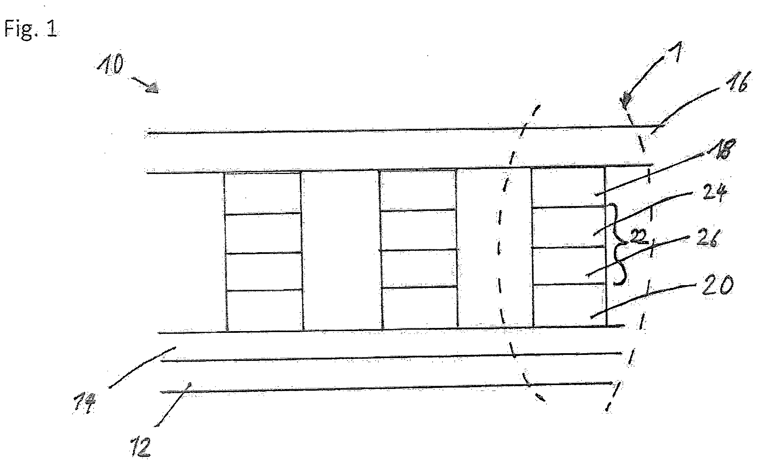

Figur 1 zeigt eine erste Ausführungsform eines elektronischen Bauelements in einer Schnittdarstellung von der Seite.

Das in Figur 1 dargestellte elektronische Bauelement 10 ist auf einem äu-ßeren Substrat 12 angeordnet, welches beispielsweise ein Wafer sein kann, der an seiner den übrigen Komponenten des elektronischen Bauelements 10 zugewandten Seite mit einem Isolator 14 versehen wurde. Das äußere Substrat 12 besteht aus den oben beschriebenen Materialien und ist beispielsweise ausgewählt aus einem Elementhalbleiter wie Silizium (Si), Germanium (Ge), Kohlenstoff in Form von Diamant, Graphit oder Graphen, aus einem Verbindungshalbleiter, insbesondere einem II-VI Verbindungshalbleiter wie Cadmium-Selenid (CdSe), Zinksulfid (ZnS), aus einem Metall wie beispielsweise Gold, Silber, Kupfer, Aluminium, Magnesium oder aus einem leitfähigem oxidischen Material wie Indiumzinnoxid (ITO), Indium-Galliumoxid (IGO), Indium-Gallium-Zinkoxid (IGZO), Aluminiumdotiertes Zinkoxid (AZO) oder fluordotiertes Zinnoxid (FTO). Bevorzugt wird kristallines Silizium als Substrat verwendet, wobei Siliziumwafer mit (100) Oberfläche besonders bevorzugt werden. Siliziumwafer, deren Oberfläche (100) orientiert ist, werden in der Mikroelektronik als übliches Substrat eingesetzt und sind in hoher Qualität und mit geringen Oberflächendefekten verfügbar.

Der Isolator 14 kann beispielsweise ein Oxid sein, wobei dieses zum Beispiel bei Verwendung eines Siliziumsubstrats mittels Ionenimplantation von Sauerstoff-Ionen in das Substrat erhalten werden kann. Auf den Isolator sind die ersten Elektroden 20 angeordnet, die in der Ausführungsform der Figur 1 in Form von Leiterbahnen ausgeführt sind, die senkrecht zur Zeichenebene verlaufen. In der in Figur 1 dargestellten Ausführungsform sind die ersten Elektroden 20 als metallische Elektroden ausgeführt. Auf den ersten Elektroden 20 sind Dioden 22 angeordnet, die beispielsweise als Zener-Dioden ausgeführt sind und jeweils eine hoch p-dotierte Schicht 26 und eine hoch n-dotierte Schicht 24 umfassen. Am Übergang der p-dotierten Schicht 26 zur n-dotierten Schicht 24 bildet sich ein pn Übergang der Diode 22 aus.

Auf der den ersten Elektroden 20 abgewandten Seite der Diode 22, welche in dieser Ausführungsform der Erfindung das erfindungsgemäße Substrat bildet, ist die molekulare Schicht 18 angeordnet. Die molekulare Schicht 18 ist bevorzugt als molekulare Monoschicht ausgeführt und damit genau eine Moleküllage dick.

Auf der der Diode 22 abgewandten Seite der molekularen Schicht 18 ist eine zweite Elektrode 16 (Gegenelektrode) angeordnet, die wie die erste Elektrode 20 als Leiterbahn ausgeführt ist. Die zweite Elektrode 16 ist gegenüber der ersten Elektrode 20 jedoch um 90° gedreht, so dass eine kreuzförmige Anordnung entsteht. Diese Anordnung wird auch Crossbar-Array (Kreuzdrahteinrichtung) genannt, wobei der 90° Winkel hier als ein Beispiel gewählt ist und auch vom rechten Winkel abweichende Anordnungen denkbar sind, bei denen sich zweite Elektroden 16 und erste Elektroden 20 kreuzen. An jeder Kreuzungsstelle zwischen einer zweiten Elektrode 16 und einer ersten Elektrode 20 ist ein Schaltelement 1 angeordnet, welches aus einem Schichtsystem mit in dieser Reihenfolge zweiten Elektrode 16, molekularer Schicht 18 und ersten Elektrode 20 gebildet wird. In der in Figur 1 dargestellten Ausführungsform ist jedem Schaltelement 1 auch eine Diode 22 zugeordnet.

Durch das Crossbar-Array kann jedes Schaltelement 1 elektrisch angesteuert werden, indem eine Spannung zwischen der korrespondierenden ersten Elektrode 20 und zweiten Elektrode 16 angelegt wird. Über die Dioden 22 wird dabei verhindert, dass Leckströme über benachbarte Schaltemente 1 fließen können.

Aufgrund der bipolaren Schaltcharakteristik der Schaltelemente 1 müssen die Dioden 22 eine nichtlineare Charakteristik für beide Polaritäten aufweisen. Hierzu werden die Dioden 22 beispielsweise als Zener-Dioden ausgeführt, wobei dazu sowohl die p-dotierte Schicht 26 als auch die n-dotierte Schicht 24 hoch dotiert werden.

Die Herstellung der Strukturen der Elektroden 16, 20 kann mittels dem Fachmann aus der Mikroelektronik bekannten Strukurierungsverfahren erfolgen. Beispielsweise kann zur Herstellung der ersten Elektroden 20 ein Lithographieverfahren eingesetzt werden. Dabei wird eine Metallschicht auf den Isolator 14 mittels Aufdampfen aufgebracht. Anschließend wird die Metallschicht mit einem Photoresist belackt, der mit den herzustellenden Strukturen belichtet wird. Nach dem Entwickeln und eventuellem Ausbacken des Resists werden die nicht benötigten Teile der Metallschicht beispielsweise durch nasschemisches Ätzen entfernt. Anschließend wird der restliche Resist beispielsweise mit einem Lösungsmittel abgetragen.

Die Strukturen der zweiten Elektroden 16 können auch mit einem Druckverfahren hergestellt werden, bei dem ähnlich wie beim konventionellen Druck ein leitfähiges Material auf das Bauelement 10 bzw. auf die molekularen Schicht 18 aufgetragen wird. Hierzu sind beispielsweise leitfähige Polymere wie Poly(3,4-ethylenedioxythiophen)/Polystyrolsulfonat (PEDOT:PSS) geeignet.

Eine weitere Möglichkeit zur Herstellung der Elektroden 16, 20, insbesondere der zweiten Elektroden 16, ist das Aufdampfen mit Hilfe einer Schattenmaske. Dabei wird eine Maske, deren Öffnungen der Form der herzustellenden Elektroden 16, 20 entsprechen, auf das Bauelement 10 aufgelegt und anschließend ein Metall aufgedampft. Der Metalldampf kann sich nur in den Bereichen, die nicht von der Maske verdeckt wurden, auf dem Bauelement 10 niederschlagen und die Elektroden 16, 20 ausbilden.

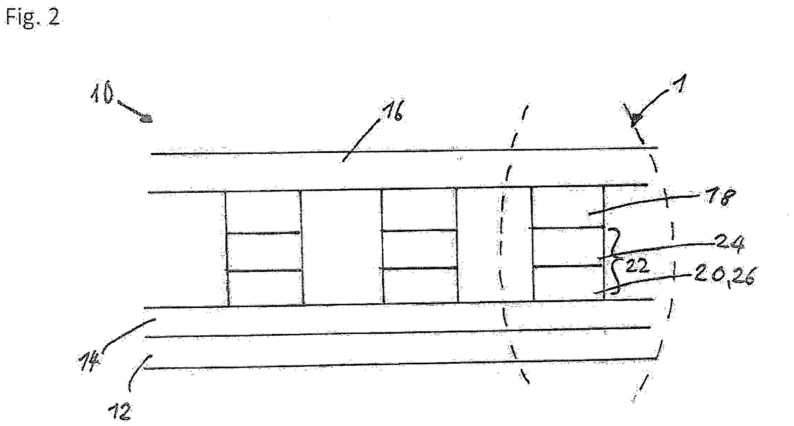

In Figur 2 ist eine weitere Ausführungsform des elektronischen Bauelements 10 dargestellt. In der Ausführungsform der Figur 2 bestehen die ersten Elektroden 20 aus einem Halbleitermaterial, welches dotiert wird, um gleichzeitig als ein Teil der Diode 22 zu fungieren. Diese Ausführungsform ist insbesondere bei Verwendung von Silicon-on-Insulator Wafern als äu-ßeres Substrat 12 vorteilhaft. Ein Silicon-on-Insulator Wafer umfasst einen Schichtaufbau, wobei in dieser Reihenfolge Schichten aus Silizium, Siliziumdioxid und stark dotiertem Silizium angeordnet sind. Ein solches Substrat kann beispielsweise hergestellt werden, indem zunächst mittels Ionenimplantation Sauerstoff-Ionen in einer Tiefe zwischen 100 nm und 10µm in das Siliziumsubstrat implantiert werden. Dicht an der Oberfläche werden Dotieratome implantiert, um eine p-Leitung oder n-Leitung einzustellen. Nach anschließender Wärmebehandlung entsteht dann in der Tiefe eine Schicht aus Siliziumdioxid, da sich die implantierten Sauerstoff-Ionen mit dem Silizium verbinden. Das Silizium wird als Substrat 10 verwendet, während die Siliziumdioxidschicht als Isolator 14 dient. Aus der dotierten Siliziumschicht werden die ersten Elektroden 20 mittels üblicher aus der Mikroelektronik grundsätzlich bekannter Strukturierungsverfahren hergestellt. In Fortbildung dieses Verfahrens kann auch direkt ein pn-Übergang an der Oberfläche des Silicon-on-Insulator Wafers erzeugt werden. Dazu werden mehrere Ionenimplantationsschritte durchgeführt, wobei in einem ersten Schritt über eine Volumenimplantation beispielsweise eine p-dotierte Schicht erzeugt wird und nachfolgend mit einer flachen, oberflächlichen Implantation eine n-dotierte Schicht erzeugt wird.

Auf den ersten Elektroden 20, die auch als Teil der Diode 22 fungieren, ist in der Ausführungsform der Figur 2 eine n-dotierte Schicht 24 angeordnet. Die ersten Elektroden 20 sind somit p-dotiert, um zusammen mit der n-dotierten Schicht 24 die Diode 22 mit einem pn-Übergang auszubilden. In dieser Ausführungsform bildet die n-dotierte Schicht 24 das erfindungsgemäße Substrat.

Die weiteren Schichten sind wie bereits zu Figur 1 beschrieben angeordnet, wobei wiederum jeweils ein Schaltelement 1 an einem Kreuzungspunkt einer ersten Elektrode 20 und einer zweiten Elektrode 16 ausgebildet wird.

Die Erfindung ist nicht auf die hier beschriebenen Ausführungsbeispiele und die darin hervorgehobenen Aspekte beschränkt. Vielmehr sind innerhalb des durch die Ansprüche angegebenen Bereichs eine Vielzahl von Abwandlungen möglich, die im Rahmen fachmännischen Handelns liegen.

Die Erfindung wird durch die Beispiele näher erläutert, ohne sie dadurch einzuschränken.

### Eingesetzte Materialien

a) Cyanoolefine, wie 1-Cyanohex-5-en sind kommerziell erhältlich, z.B. von Sigma-Aldrich.
b) N-Allyl-N'-methylimidazolium-trifluormethylsulfonylimid (amim-NTf₂) ist kommerziell erhältlich, beispielsweise von der Merck KGaA oder Sigma-Aldrich.
c) N-Allyl-N'-methylimidazolium-tetraphenylborat (amim-BPh₄): 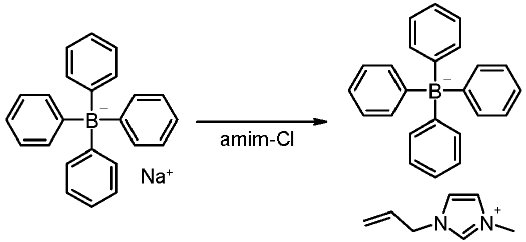 Eine Lösung von amim-Cl (490 mg, 3.0 mmol) in Wasser (10 mL) gab man tropfenweise zu einer Lösung von Natrium-tetraphenylborat (1.0 g, 2.9 mmol) in Wasser (12 mL). Die sich dabei bildende farblose Suspension wurde 2 h bei RT gerührt und anschließend filtriert. Der farblose feste Rückstand wurde mit kaltem Wasser und Methanol gründlich gewaschen. Man erhielt 3 g Rohprodukt, das aus THF/H₂O 1:2 (40 mL) kristallisiert wurde: farblose Kristalle von amim-BPh₄ (1.0 g, 77%). Fp. 151°C; ¹H NMR (400 MHz, THF-*d*₈, 298 K): δ = 3.21 (s, 3H, im⁺-C*H*₃), 4.19 (dt, *J* = 6.1 Hz, *J* = 1.4 Hz, 2H, im⁺-C*H*₂), 5.17 (d, *J =* 17.0 Hz, 1H, =C*H*-H), 5.32 (d, *J =* 10.2 Hz, 1H, =C*H*-H), 5.65-5.86 (m, 1H, -C*H*=CH₂), 6.35 (d, *J* = 1.7 Hz, 1H, im⁺-*H*), 6.67-6.79 (m, 4H, ar-4-*H*), 6.89-6.93 (m, 9H, ar-2-H, im⁺-*H*), 6.97 (t, *J =* 1.9 Hz, 1H, im⁺-*H*), 7.42 (m, 8H, ar-3-H); ¹³C NMR (100 MHz, THF-*d*₈, 298 K): δ = 35.2, 51.2, 120.1, 121.2, 121.6, 123.3, 125.2, 130.7, 135.7, 136.0, 164.2 (quart, *J*_{CB} = 49.3 Hz, BPh₄⁻-1-*C*); ¹¹B NMR (128 MHz, THF-*d*₈, 298 K): δ = -6.41 (d, *J* = 3.9 Hz).
d) N-Allyl-N'-methylimidazolium-(3,4,5-trifluorphenyl)triphenylborat (amim-B(PhF₃)Ph₃):

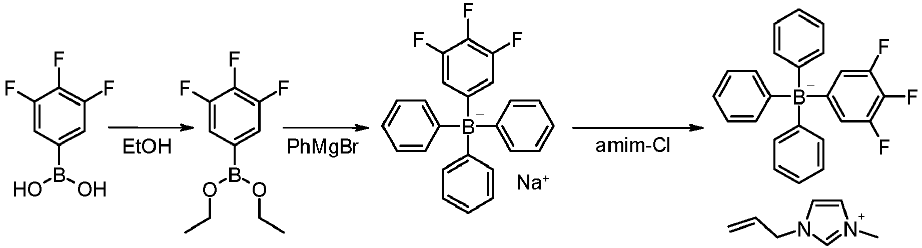

*3,4,5-Trifluorbenzolboronsäure-Diethylester* Eine Mischung von 3,4,5-Trifluorbenzolboronsäure (17.9 g, 0.1 mol), CHCl₃ (250 mL) und Ethanol (130 mL) wurde für 48 h unter Argonatmosphäre über einen Soxhlet-Extraktor zum Rückfluss erhitzt, der mit Molekularsieb 0.4 nm (2 mm ∅ Perlen, 72 g) beladen war. Die Lösung wurde im Vakuum zur Trockene eingedampft, und das dabei anfallende Öl im Kugelrohr bei ca. 90°C und 0.02 mbar destilliert. Das so erhaltene farblose Öl (15 g, 65%) wurde ohne weitere Aufreinigung für die nächste Stufe verwendet. MS (EI): *m*/*z* (%) = 232 [M⁺] (40), 159 (100), 145 (35), 100 (50), 73 (65), 45 (90).

*Natrium-(3,4,5-trifluorphenyl)triphenylborat* (Na⁺ B(PhF₃)Ph₃⁻): 1 M Phenylmagnesiumbromid in THF (100 mL, 100 mmol) wurde tropfenweise zu einer Lösung von 3,4,5-Trifluorbenzolboronsäure-Diethylester (6.5 g, 28 mmol) in THF (250 mL) hinzugefügt. Die Mischung wurde 5 h bei 60°C gerührt. Nach Abkühlen auf RT gab man tropfenweise 2 M wässrige Na₂CO₃-Lösung (200 mL) zu. Nach Zugabe von THF (450 mL) wurde die organische Schicht abgetrennt. Die verbleibende wässrige Phase wurde noch einmal mit THF (100 mL) extrahiert. Die vereinten organischen Phasen wurden zur Trockene eingedampft und das so erhaltene gelbe Öl wurde in Diethylether (500 mL) aufgenommen, über Na₂SO₄ getrocknet und filtriert. Nach Zugabe von Toluol (500 mL) wurde die Lösung auf 50 mL eingeengt, worauf farblose Kristalle ausfielen. Nach Abkühlen auf 5°C wurden die Kristalle abfiltriert und zuerst mit kaltem Toluol, dann mit *n*-Pentan gewaschen und getrocknet: Na⁺ B(PhF₃)Ph₃⁻ (8.8 g, 79%) als farblose Kristalle; ¹H NMR (400 MHz, THF-*d*₈, 298 K): δ = 6.73-6.77 (mc, 3H, ar-*H*), 6.83-6.90 (m, 8H, ar-*H*), 7.20 (br. m, 6H, ar-*H*); ¹⁹F NMR (376 MHz, THF-*d*₈, 298 K): δ = -143.67 (dd, *J* = 20.4 Hz, *J* = 11.7 Hz, 2F), -174.81 (tt, *J* = 20.4, *J* = 7.8 Hz, 1F); ¹¹B NMR (128 MHz, THF-*d*₈, 298 K): δ = -6.56 (s); MS (FIA-APCI, negative ion mode): *m*/*z* (%) = 373 [C₂₄H₁₇BF₃⁻] (100%).

N-Allyl-N'-methylimidazolium-(3,4,5-trifluorophenyl)triphenylborat (amim⁺ B(PhF₃)Ph₃⁻): Man tropfte eine Lösung von amim-CI (4.00 g, 25.2 mmol) in Wasser (100 mL) unter Rühren zu einer Lösung von Na⁺ B(PhF₃)Ph₃⁻ (8.80 g, 22.2 mmol) in Wasser (200 mL). Nach 1 h Rühren bildete die weißliche Emulsion einen klebrigen Belag auf dem Rührer. Man goss den wässrigen Überstand ab und digerierte den Rückstand in MTBE (Methyl-tert.-butylether) (100 mL). Der nun feste Rückstand wurde abfiltriert und mit eiskaltem MTBE gewaschen. Wasserreste wurden durch azeotropes Trocknen mit Toluol entfernt. Das Rohprodukt (9 g) wurde in MTBE (50 mL) dispergiert, 10 min bei RT gerührt, abfiltriert und im Vakuum bei 38°C getrocknet und ergab 7.0 g (63%) eines weißlichen Pulvers (Reinheit 99.1%; HPLC: RP-18 Purospher 250-4 1x 52a, CH₃CN/0.1M wässr. KH₂PO₄ 60:40). Fp. 105°C; ¹H NMR (400 MHz, THF-*d*₈): δ = 3.38 (s, 3H, im⁺-C*H*₃), 4.34 (dt, *J* = 6.1 Hz, *J =* 1.4 Hz, 2H, im⁺-C*H*₂CH=), 5.18 (dq, *J =* 17.0 Hz, *J* = 1.3 Hz, 1H, =CH-H), 5.31 (dq, *J* = 10.3 Hz, *J* = 1.2 Hz, 1H, =C*H*-H), 5.79 (ddt, *J* = 16.5 Hz, *J* = 10.2 Hz, *J* = 6.1 Hz, 1H, C*H*=CH₂), 6.71- 6.79 (m, 3H), 6.81-6.97 (m, 8*H*), 7.01 (mc, 1H, im⁺-*H*), 7.07 (mc, 1H, im⁺-*H*), 7.28 (mc, 6H, ar-*H*), 7.36 (mc, 1H, im⁺-*H*); ¹⁹F NMR (376 MHz, THF-*d*₈): δ = - 142.87 (dd, *J* = 20.4 Hz, J = 11.5 Hz, 2F, ar-3-*F*), -174.2 (tt, *J* = 20.3 Hz, J = 7.7 Hz, 1F, ar-4-*F*); ¹¹B NMR (128 MHz, THF-*d*₈): δ = -6.54; MS (FIA-APCI, negative ion mode): *m*/*z* (%) = 373 [C₂₄H₁₇BF₃⁻] (100%).

### Allgemeine Derivatisierungsvorschriften zur Herstellung beschichteter Substrate

Im Prinzip erfolgt die Derivatisierung von Siliziumoberflächen analog zu O. Seitz et al., Langmuir 22 (2006), 6915-6922. Das Siliziumsubstrat wird zunächst mit Aceton im Ultraschallbad von organischen Verunreinigungen gereinigt und dann mit Piranha (konz. H₂SO₄/30% H₂O₂ 70:30) behandelt. Nach dem Spülen mit Wasser wird das Substrat unter Sauerstoffausschluss mit wässriger NH₄F-Lösung behandelt und anschließend mit sauerstofffreiem Wasser gewaschen. Das nun wasserstoffterminierte Substrat wird für 12 h bei 120°C unter strengem Sauerstoffausschluss mit dem reinen Derivatisierungsreagenz oder einer 10%igen Lösung desselben in 1,2-Dichlorbenzol behandelt. Anschließend wäscht man das derivatisierte Substrat mit Aceton im Ultraschallbad, spült mit Isopropanol nach und trocknet in staubfreier Umgebung mit einem Stickstoffstrahl.
***a)*** ***Direkte Derivatisierung ohne Lösungsmittel:*** Ein frisch hergestellter, Wasserstoff-terminierter Chip (8 mm x 8 mm x 575±25 µm, 100-Orientierung, hochgradig Bor-dotiert) wurde in einem mit Argon gespültem Schlenk-Gefäß mit entgastem Derivatisierungsreagenz (z. B. 6-Cyanohex-1-en) für 18 h auf 110°C erhitzt. Der nun organomodifizierte Chip wurde aus dem Reaktionsgefäß entnommen, 5 min mit Aceton im Ultraschallbad gespült, mit Aceton und Isopropanol nachgespült und im Stickstoffstrom getrocknet. Der derivatisierte Chip wird in einem Eppendorf-Gefäß aufbewahrt.
***b) Derivatisierung mit Lösung:*** analog zu a) wurde eine 10%ige (w/w) Lösung des Derivatisierungsreagenz in 1,2-Dichlorbenzol verwendet. Diese Variante wurde für alle bei RT nicht flüssigen Derivatisierungsreagenzien verwendet.

### Topographische und elektrische Charakterisierung

Ein memristives Schaltverhalten wurde für mehrere, dipolare Monoschichtsysteme gemessen, so dass diese als erfindungsgemäße Ausführungsbeispiele verifiziert werden konnten. Alle Schichten wurden auf *p*+ Si (100)-Substraten präpariert. Es wurden dabei die in der zweiten Spalte angegebenen organischen Gruppen als Monolagen erzielt, wobei dafür die in der dritten Spalte angegebenen Vorläuferverbindungen eingesetzt wurden (Precursors).

| Beispiele | Monolage | Precursor | Abkürzung |
|---|---|---|---|
| Beispiel 1 | 1-Cyanohex-6-yl | 1-Cyanohex-5-en | C6CN |
| Beispiel 2 | N-Prop-3-yl-N'-methylimidazoliniumtrifluormethylsulfonylimid | N-Allyl-N'-methylimidazoliniumtrifluormethylsulfonylimid | Si-IL-1 |
| Beispiel 3 | N-Prop-3-yl-N'-methylimidazolinium-(3,4,5-trifluorphenyl)-triphenylborat | N-Allyl-N'-methylimidazolinium-(3,4,5-trifluorphenyl)-triphenylborat | Si-IL-3 |
| VergleichsBeispiel | Oct-1-yl | Oct-1-en | C8 |

Vor Durchführung der elektrischen Messungen wurden die unterschiedlichen Schichten eingehend bezüglich ihrer topographischen Beschaffenheit (Schichtdicke, Rauigkeit, etc.) charakterisiert. Hierbei ergaben Ellipsometrie-Messungen folgende Schichtdicken: C8: 1,3-1,4 nm, C6CN: 2,0-2,3 nm. Diese liegen um einen Faktor 1,2-2,1 über den theoretischen Moleküllängen. Die Dicken der ionischen Doppellagensysteme waren 1,3-1,4 nm (Si-IL-1) bzw. 1,8-2,1 nm (Si-IL-3). Sie lagen damit um einen Faktor 1,3-1,5 über den theoretischen Werten. Beide als zu groß erhaltenen Schichtdicken (gegenüber dem Erwartungswert für Monolagen) können mit lokaler Rauigkeit, geringfügiger Cluster- bzw. Multilagenbildung erklärt werden. Die Rauigkeit (root-mean- square (rms), über 5x5 µm²) der Schichtsysteme wurde mittels Rasterkraftmikroskopie (AFM) bestimmt. Sie lag für alle untersuchten Schichten im Bereich 0,3-2,8 nm. Die untere Grenze dieser Rauigkeiten ist vergleichbar mit der Rauigkeit der polierten Si-Substrate, d.h. die Monolagen bilden die Oberfläche i.a. konform ab. Örtlich auftretende Partikel (evtl. Molekülcluster) mit Größen im Bereich bis zu einigen 10 nm hingegen führen zu entsprechend größeren rms-Werten, da diese auf einer Mittelung über den gesamten Scan-Bereich beruhen.

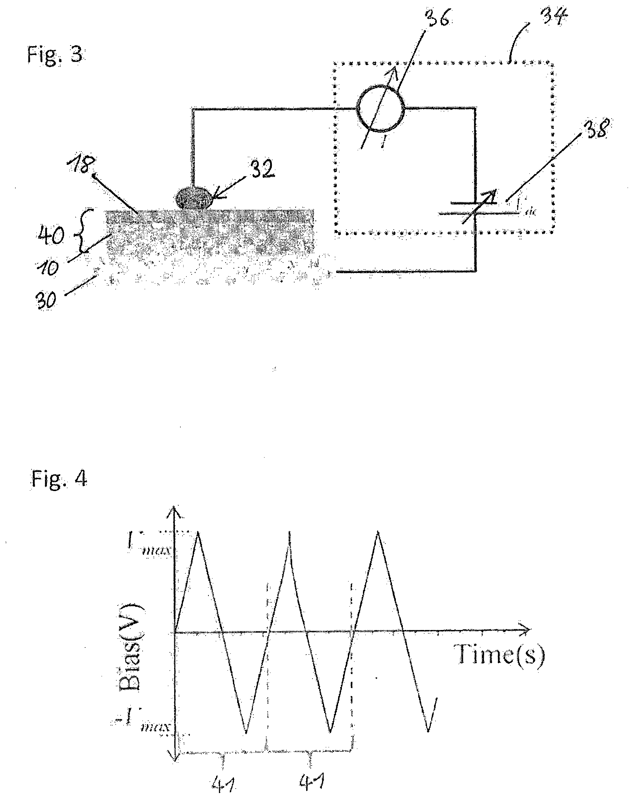

Die elektrischen Messungen an verschiedenen Proben werden nachfolgend mit Bezug zu den Figuren 3 bis 10 beschrieben.

Figur 3 zeigt einen Versuchsaufbau zur Charakterisierung der elektrischen Eigenschaften einer molekularen Schicht auf einem Substrat.

In Figur 3 ist schematisch dargestellt, wie die elektrischen Eigenschaften einer Probe 40 ermittelt werden. Die Probe 40 umfasst das Substrat 10, auf das eine molekulare Schicht 18 aufgebracht wurde. Das Substrat 10 ist elektrisch leitend und dient hierbei als eine Elektrode, um die molekulare Schicht 18 elektrisch zu kontaktieren. Die elektrische Verbindung zu einem Messgerät 34, wird dabei über eine bewegliche Kupferplatte 30 hergestellt. Die Probe 40 wird dazu auf der Kupferplatte 30 platziert und kann durch Bewegen der Kupferplatte 30 relativ zu einer weiteren Elektrode bewegt werden. Als weitere Elektrode zur elektrischen Kontaktierung der Oberseite der molekularen Schicht 18 dient ein hängender Quecksilbertropfen 32, der über seine Aufhängung ebenfalls mit dem Messgerät 34 in Verbindung steht. Der Durchmesser des Quecksilbertropfens 32 beträgt typischerweise etwa 150 µm.

Nach dem Platzieren der Probe 40 auf der Kupferplatte 30 wird diese in Relation zum Quecksilbertropfen 32 so bewegt, dass der Quecksilbertropfen 32 die Oberfläche der molekularen Schicht 18 berührt. Dies ermöglicht eine zerstörungsfreie und wechselwirkungsfreie Prüfung der elektrischen Leitfähigkeitseigenschaften der Probe 40.

Für die elektrischen Messungen ist das Messgerät 34 bevorzugt als Source-Measure Unit ausgeführt, das heißt das Messgerät 34 stellt über eine Spannungsquelle 38 eine Ausgangspannung bereit und misst gleichzeitig über eine Strommesseinheit 36 den resultierenden elektrischen Strom.

Für die Messungen wird zwischen der Kupferplatte 30 und dem Quecksilbertropfen 32 eine elektrische Spannung angelegt und variiert, gleichzeitig wird der elektrische Strom durch die Probe 40 gemessen. Dabei variiert die Spannung zyklisch zwischen einem vorgegebenen Maximalwert Vₘₐₓ und einem vorgegebenen Minimalwert Vₘᵢₙ, wie in Figur 4 dargestellt. Für die Versuche wurde eine Source-Measure-Unit vom Typ Keithley 2635 eingesetzt.

Figur 4 zeigt beispielhaft mehrere Zyklen, in denen die angelegte Spannung zyklisch zwischen Vₘₐₓ und Vₘᵢₙ variiert wird, wobei ein sägezahnförmiger Spannungsverlauf entsteht.

An die beiden Elektroden (das Substrat 10 und der Quecksilbertropfen 32, vergleiche Figur 3) wird über das Messgerät 34 eine Gleichspannung angelegt, welche über die Zeit in einer zyklischen Abfolge zwischen einem maximalen negativen und maximalen positiven Spannungswert bei konstanter Rate, beispielsweise 5 mV/s variiert. In der Figur 4 sind drei solcher Zyklen 41 eingezeichnet. Der resultierende Strom durch die zu charakterisierende Probe 10 wird gemessen und aufgezeichnet.

Die aufgezeichneten Ströme für verschiedene Proben werden in den folgenden Figuren dargestellt und in der dazugehörigen Beschreibung näher erläutert.

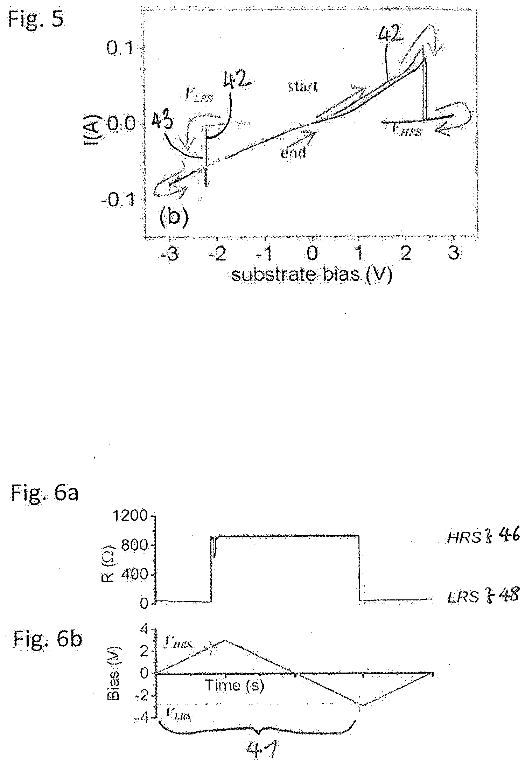

Figur 5 zeigt den aufgezeichneten Strom durch eine Probe mit dem Monolagensystem C6CN.

Der Widerstand der Probe schaltet bei einer charakteristischen Schaltspannung V_{HRS} abrupt von einem Zustand mit geringem Widerstand (Low Resistance State, LRS) in einen Zustand mit hohem Widerstand (High Resistance State, HRS). Für eine charakteristische (negative) erste Schaltspannung V_{LRS} schaltet das System ebenso abrupt reversibel zurück in den LRS. Dieses Schaltverhalten ist anhand der zeitlichen Abfolge der Widerstandswerte für einen ausgewählten Zyklus in Figur 6a dargestellt.

Figur 6a zeigt die zeitliche Abfolge der Widerstandswerte der Probe mit dem Monolagensystem C6CN für einen ausgewählten Zyklus 41. In Figur 6b ist das angelegte Spannungsprofil für diesen Zyklus 41 dargestellt.

Wie den Figuren 6a und 6b entnommen werden kann, schaltet der Widerstand der Probe bei der (positiven) zweiten Schaltspannung V_{HRS} aus dem Zustand mit geringem Widerstand LRS, der in der Figur 6a mit dem Bezugszeichen 48 gekennzeichnet ist, in den Zustand mit hohem Widerstand HRS, der in der Figur 6a mit dem Bezugszeichen 46 gekennzeichnet ist. Der dazugehörige Spannungsverlauf für den Zyklus 41 ist in der Figur 6b dargestellt.

Das Schaltverhalten konnte für sieben Testzyklen reproduziert werden, ohne dass eine Degradation des Effekts bzw. der Probe auftrat. Das OFF-ON Verhältnis der beiden Widerstandswerte R_{HRS}:R_{LRS} betrug ca. 6500 (bei einer Auslesespannung von 0,1V). Die zeitliche Stabilität beider Zustände wurde durch einmaliges Halten der Spannung bei 0V (für den LRS) bzw. bei -1,5V (HRS) getestet: für eine Haltezeit von jeweils 15 min. verblieb das System stabil im jeweiligen Zustand.

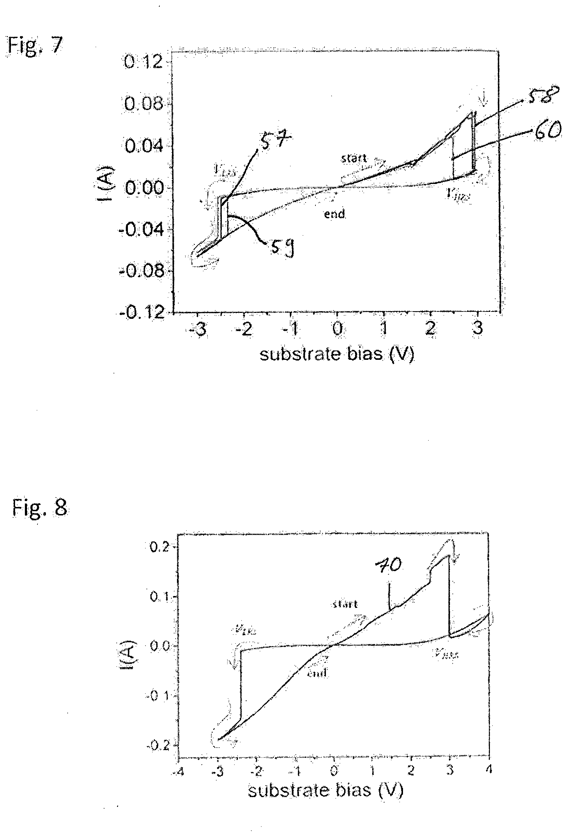

Figur 7 zeigt den aufgezeichneten Strom durch eine Probe mit dem Schichtsystem Si-IL-1.

Das Monolagensystem Si-IL-1 zeigt ein qualitativ zu C6CN ähnliches Schaltverhalten. Nach einem initialen Schalten von HRS nach LRS bei negativer Substratspannung wurden sich wiederholende Schaltzyklen durchlaufen. Im Gegensatz zu C6CN waren jedoch für das erste Schalten aus dem Zustand mit geringem Widerstand (LRS) in den Zustand mit hohem Widerstand (HRS) größere positive Spannungen erforderlich; diese Schaltspannung V_{HRS} variierte für einige Proben stetig zu geringeren Werten mit fortlaufenden Zyklen. In der Figur 7 sind die Zyklen 7-10 einer Probe mit fast unverändertem, stabilem Schaltverhalten dargestellt. Die Zyklen 7 bis 10 sind in der Figur 7 mit den Bezugszeichen 57, 58, 59 und 60 gekennzeichnet. Auch für dieses Monolagensystem wurden Haltemessungen (z.B. bei -1,2 V im Zustand HRS, nach dem 8. Zyklus) durchgeführt, ohne messbare Variation des Widerstandswerts während des stationären Haltens der Spannung. Das Widerstandsverhältnis R_{HRS}/R_{LRS} erreichte typisch Werte von >1000 bei einer Auslesespannung von 0,1 V.

Für das gemessene, reproduzierbare Schaltverhalten ist die Präparation homogen dünner, vorzugsweise monomolekularer Schichten ausschlaggebend. Dies konnte mit Hilfe von Vergleichsmessungen an einer Probe desselben Systems (Si-IL-1) nachgewiesen werden, welche jedoch aufgrund einer anderen Präparationsmethodik eine größere Schichtdicke (2,5 nm) und ausgeprägte Ausbildung von Multilagen bzw. Clusterstrukturen aufwies. Für dieses System wurde zwar ein Schaltverhalten von HRS nach LRS bei ca. V_{LRS} = -2,8 V beobachtet, jedoch konnte kein Zurückschalten nach HRS bis zu +3,0 V erreicht werden. Die Kennlinie wies stattdessen signifikante Instabilitäten (Mehrfachsprünge) des Stromwertes auf.

Figur 8 zeigt den aufgezeichneten Strom durch eine Probe mit Schichtsystem Si-IL-3.

Ein zu den dünnen, d.h. näherungsweise monomolekularen, Si-IL-1-Schichten qualitativ ähnliches Schaltverhalten wurde auch für eine Probe mit dem Schichtsystem Si-IL-3 gemessen. Dargestellt ist ein beispielhaft ausgewählter Zyklus 70, bei dem ein Schaltverhalten des Widerstands bei den Spannungen V_{HRS} und V_{LRS} beobachtet wird. Die Schichtdicken der untersuchten Lagen variierten geringfügig im Bereich 1,8-2,1 nm. Auch hier wurde ein offenbar vorhandener Einfluss der Schichtdicke auf das Schwellenverhalten des Schaltprozesses bestätigt: während bei 1,8 nm dicken Schichten eine Ausbeute von rund 40% der untersuchten Positionen auf dem Testchip ein zu Figur 8 qualitativ ähnliches Schaltverhalten zeigten, konnte für dickere Molekülschichten (2,1 nm) ein (zudem instabiles) Schaltverhalten nur für wenige ausgewählte Positionen beobachtet werden. Insgesamt waren Reproduzierbarkeit und Stabilität für das System Si-IL-3 geringer als für die beiden Systeme C6CN und Si-IL-1.

### Kontrollmessungen

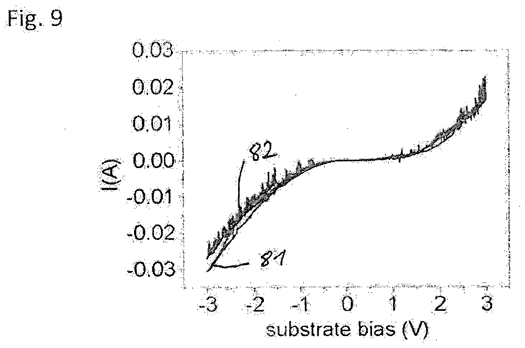

Figur 9 zeigt den aufgezeichneten Strom für zwei Zyklen der I-U-Charakteristik einer Octyl-funktionalisierten ("C8") Si-Probe.

Die für die Erfindung zentrale Voraussetzung eines ausgeprägten Dipolmoments der Moleküle der Monolagen konnte durch Kontrollmessungen an nicht organisch beschichteten bzw. mit nur einer einfachen Octyl-Monolage (ohne CN-Endgruppe) beschichteten Silizium-Substraten nachgewiesen werden: in beiden Fällen wurde kein Schalt- bzw. hysteretisches Verhalten der gemessenen Widerstände beobachtet. In der Figur 9 sind dazu exemplarisch zwei Zyklen 81 und 82 für eine C8-Alkan-Monolage dargestellt. Ein Schaltverhalten des elektrischen Widerstands ist in den beiden Zyklen 81 und 82 nicht erkennbar.

### Elektrische Messungen mit permanenter zweiter Elektrode

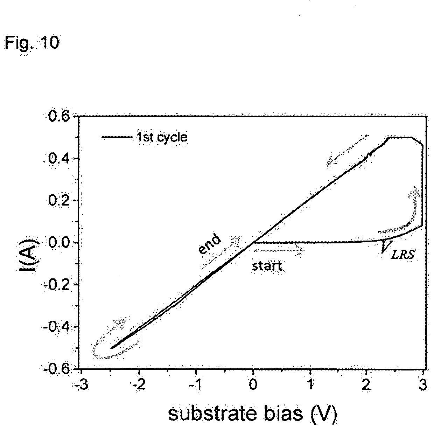

Per Schattenbedampfungstechnik wurden anstelle der Hg-Elektrode permanente, dünne Metallfilme aus Pb (250 nm) mit nachfolgend Ag (60 nm) auf eine Si-IL-3-Probe aufgebracht. Die so präparierten oberen Elektroden wurden mit einer Messnadel kontaktiert. Im Übrigen verliefen die elektrischen Messungen analog zur oben beschriebenen Anordnung bzw. Vorgehensweise. Messungen bei kleinen Spannungen bis 1V zeigten kein Schaltverhalten. Das System verblieb im Ausgangszustand (OFF), und die gemessenen Ströme skalierten korrekt mit der variierten Fläche der Metallelektrode. Für höhere Spannungen konnte ein einmaliges Schalten in den ON-Zustand beobachtet werden, bei einem Widerstandsverhältnis von -2000, bei 0.1 V Auslesespannung (s. Fig. 10). Systeme mit derartigem, einmaligem Schaltverhalten sind für Anwendungen im Bereich von PROMs (programmable read-only memory) relevant.

### Bezugszeichenliste

- 1: Schaltelement
- 10: elektronisches Bauelement (mit Kreuzdrahtvorrichtung)
- 12: äußeres Substrat
- 14: Isolator
- 16: zweite Elektrode
- 18: molekulare Schicht
- 20: erste Elektrode
- 22: Diode
- 24: n⁺-Schicht
- 26: p⁺-Schicht
- 30: Kupferplatte
- 32: Hg-Tropfen
- 34: Messgerät
- 36: Strommesseinlheit
- 38: Spannungsquelle
- 40: Probe
- 41: Messzyklus
- 42: 2. Zyklus
- 43: 3. Zyklus
- 44: 4. Zyklus
- 46: hoher Widerstand
- 48: geringer Widerstand
- 57: Zyklus 7
- 58: Zyklus 8
- 59: Zyklus 9
- 60: Zyklus 10
- 70: Zyklus
- 81: 1. Zyklus
- 82: 2. Zyklus

## Patentansprüche

1. Elektronisches Bauelement (10) umfassend mehrere Schaltelemente (1), welche in dieser Reihenfolge
eine erste Elektrode (20),
eine molekulare Schicht (18), gebunden an ein Substrat, und
eine zweite Elektrode (16) umfassen,
wobei die erste Elektrode (20) das Substrat für die molekulare Schicht darstellt,
**dadurch gekennzeichnet, daß** die molekulare Schicht im Wesentlichen aus Molekülen (M) besteht, die eine Verbindungsgruppe (V) und eine Endgruppe (E) mit einer polaren oder ionischen Funktion enthalten und wobei die Moleküle (M) eine oder mehrere Verbindungen der Formel (I) darstellen,
- ~ (A)ₛ-(V)-[Y¹-(Z¹-Y²)ₘ]ₜ-(E) (I)
worin
A eine Ankergruppe;
V eine C₁-C₂₅-Alkylengruppe, die in der Kette ein oder mehrere funktionelle Gruppen und/oder ein oder mehrere 3-bis 6-gliedrige, gesättigte oder teilweise ungesättigte, alicyclische oder heterocyclische Ringe enthalten kann und bei der ein oder mehrere H-Atome durch Halogen ersetzt sein können;
Y¹ und Y² jeweils unabhängig voneinander eine aromatische oder eine alicyclische Gruppe mit vorzugsweise 4 bis 25 C-Atomen bedeuten, die auch kondensierte Ringe enthalten kann und die ein- oder mehrfach mit einer Gruppe R^{L} substituiert sein kann;
R^{L} jeweils unabhängig OH, SH, SR°, -(CH₂)ₙ-OH, F, Cl, Br, I, -CN, -NO₂, -NCO, -NCS, -OCN, -SCN, -C(=O)N(R°)₂, -C(=O)R°, -N(R°)₂, -(CH₂)ₙ-N(R°)₂, gegebenenfalls substituiertes Silyl, gegebenenfalls substituiertes Aryl oder Cycloalkyl mit 6-20 C-Atomen oder lineares oder verzweigtes Alkyl, Alkoxy, Alkylcarbonyl, Alkoxycarbonyl, Alkylcarbonyloxy oder Alkoxycarbonyloxy mit 1-25 C-Atomen, in dem ein oder mehrere H-Atome durch F oder Cl ersetzt sein können und in dem zwei vicinale Gruppen R^{L} zusammen gegebenenfalls =O sein können;
n 1, 2, 3 oder 4;
Z¹ jeweils unabhängig eine Einfachbindung, -O-, -S-, -CO-, -CO-O-, -O-CO-, -O-CO-O-, -OCH₂, -CH₂O-, -SCH₂-, -CH₂S-, -CF₂O-, -OCF₂-, -CF₂S-, -SCF₂-, -(CH₂)ₙ-, -CF₂CH₂-, -CH₂CF₂₋, -(CF₂)ₙ-, -CH=CH-, -CF=CF-, -C=C-, -CH=CH-COO-, -OCO-CH=CH- oder -CR°₂;
R° jeweils unabhängig voneinander H oder C₁-C₁₂-Alkyl
m 0, 1, 2, 3, 4, oder 5,
s und t unabhängig voneinander 0 oder 1
bedeuten, und
∼die Bindung an das Substrat angibt,
und wobei die Endgruppe (E) eine polare Endgruppe ist, die mindestens eine Bindung aufweist, bei der die Elektronegativitätsdifferenz zwischen den beteiligten Atomen mindestens 0.5 beträgt, gewählt aus CN, SCN, NO₂, (C₁-C₄)-Haloalkyl, bevorzugt CF₃, (C₁-C₄)-Haloalkoxy, bevorzugt OCF₃, -S-(C₁-C₄)-Haloalkyl, bevorzugt SCF₃, S(O)₂-(C₁-C₄)-Haloalkyl, bevorzugt SO₂CF₃, SFs, OSFs, N(C₁-C₄-Haloalkyl)₂, bevorzugt N(CF₃)₂, N(CN)₂ und (C₆-C₁₂)-Haloaryl, bevorzugt Mono-, Di- oder Tri-fluorphenyl,
oder wobei die Endgruppe (E) eine redox-inaktive kationische oder anionische Gruppe ist, gewählt aus Imidazolium-, Pyridinium-, Pyrrolidinium-, Guanidinium-, Uronium-, Thiouronium-, Piperidinium-, Morpholinium-, Ammonium- und Phosphonium-Gruppen beziehungsweise Halogeniden, Boraten, Sulfonaten, Carboxylaten, Phosphaten, Phosphinaten, perfluorierten Alkyl-Sulfonaten und - Carboxylaten, Imidanionen und Amidanionen, bevorzugt gegebenenfalls substituierten Tetraphenylboraten, Tetrafluoroborat, Trifluoracetat, Triflat, Hexafluorophosphat, Phosphinaten und gegebenenfalls substituierten Tosylaten, wobei die Gegenionen der kationischen und anionischen Gruppen gewählt sind aus den aufgeführten anionischen beziehungsweise kationischen Gruppen.

2. Elektronisches Bauelement (10) gemäß Anspruch 1, wobei die Verbindungsgruppe (V) eine lineare oder verzweigte C₁-C₂₅-Alkylengruppe ist, in der eine oder mehrere nicht benachbarte CH₂-Gruppen jeweils durch -C=C-, -CH=CH-, -NR'-, -O-, -S-, -CO-, -CO-O-, -O-CO-, -O-CO-O- oder einen 3-6 gliedrigen, gesättigten oder teilweise ungesättigten, alicyclischen oder heterocyclischen Ring ersetzt sein können, wobei N, O und/oder S nicht direkt aneinander gebunden sind, in der ein oder mehrere tertiäre Kohlenstoffatome (CH-Gruppen) durch N ersetzt sein können und in der ein oder mehrere Wasserstoffatome durch Halogen ersetzt sein können, wobei R' jeweils unabhängig voneinander H oder C₁-C₁₂-Alkyl bedeutet.

3. Elektronisches Bauelement (10) gemäß Anspruch 2, wobei die Verbindungsgruppe (V) eine lineare oder verzweigte C₁-C₁₀-Alkylengruppe ist, in der eine oder mehrere nicht benachbarte CH₂-Gruppen jeweils durch -O-, -S-, oder einen 3-6-gliedrigen, gesättigten alicyclischen Ring ersetzt sein können, wobei O und/oder S nicht direkt aneinander gebunden sind, und in der ein oder mehrere Wasserstoffatome durch F und/oder Cl ersetzt sein können.

4. Elektronisches Bauelement (10) nach einem der Ansprüche 1 bis 3, wobei die Moleküle (M) neben der Verbindungsgruppe (V) und der Endgruppe (E) eine Ankergruppe (A) umfassen, über welche die Verbindungsgruppe an das Substrat gebunden ist.

5. Elektronisches Bauelement (10) nach Anspruch 4, wobei die Ankergruppe (A) über eine kovalente Bindung an das Substrat gebunden ist.

6. Elektronisches Bauelement (10) nach Anspruch 5, wobei die Ankergruppe (A) über Physisorption an das Substrat gebunden ist.

7. Elektronisches Bauelement (10) nach Anspruch 5 oder 6, wobei die Ankergruppe (A) gewählt ist aus Carboxylat-, Phosphonat-, Alkoholat-, Arylat-, bevorzugt Phenolat-, Thiolat- und Sulfonatgruppen oder Fullerenderivaten, bevorzugt [60]PCBM ([6,6]-Phenyl-C61-butansäuremethylester) und [70]PCBM ([6,6]-Phenyl-C71-butansäuremethylester).

8. Elektronisches Bauelement (10) nach einem der Ansprüche 1 bis 7, **dadurch gekennzeichnet, dass** die molekulare Schicht eine molekulare Monoschicht ist.

9. Elektronisches Bauelement (10) nach einem der Ansprüche 1 bis 8, **dadurch gekennzeichnet, dass** die erste Elektrode (20) aus einem Material besteht, gewählt aus Elementhalbleitern, bevorzugt Si, Ge, C (Diamant, Graphit, Graphen), Sn (alpha Modifikation) und Se, Verbindungshalbleitern der Gruppen III-V, bevorzugt GaAs, InAs, InP, GaSb und GaN, und Verbindungshalbleitern der Guppen II-VI, bevorzugt CdSe und ZnS, in unterschiedlicher Dotierung, Metallen, bevorzugt Au, Ag, Cu, Al und Mg, sowie leitfähigen, oxidischen Materialien, bevorzugt ITO, IGO, IGZO, AZO und FTO.

10. Elektronisches Bauelement (10) nach einem der Ansprüche 1 bis 9, **dadurch gekennzeichnet, dass** die molekulare Schicht an eine oxidische oder fluoridische Zwischenschicht gebunden ist.

11. Elektronisches Bauelement (10) nach Anspruch 10, **dadurch gekennzeichnet, dass** die oxidische oder fluoridische Zwischenschicht aus TiO₂, Al₂O₃, HfO₂, SiO₂ oder LiF gebildet wird.

12. Elektronisches Bauelement (10) nach einem der Ansprüche 1 bis 11, **dadurch gekennzeichnet, dass** die zweite Elektrode (16) aus einem leitenden oder halbleitenden Material oder einer Kombination mehrerer dieser Materialien gewählt aus Hg, In, Ga, InGa, Ag, Au, Cr, Pt, PdAu, Pb, Al, Mg, CNT, Graphen und leitfähigen Polymeren besteht.

13. Elektronisches Bauelement (10) nach einem der Ansprüche 1 bis 12, **dadurch gekennzeichnet, dass** die erste Elektrode (20) und die zweite Elektrode (16) metallische Leiter sind oder dass die zweite Elektrode (16) ein metallischer Leiter ist und die erste Elektrode (20) halbleitend ist.

14. Elektronisches Bauelement (10) nach einem der Ansprüche 1 bis 13, **dadurch gekennzeichnet, dass** zwischen der ersten Elektrode (20) und der molekularen Schicht (18) oder der zweiten Elektrode (16) und der molekularen Schicht (18) eine Diode (22) angeordnet ist.

15. Elektronisches Bauelement (10) nach Anspruch 14, **dadurch gekennzeichnet, dass** die Diode (22) eine n+ dotierte Schicht (24) und eine p+ dotierte Schicht (26) umfasst, wobei die n+ dotierte Schicht (24) oder die p+ dotierte Schicht (26) gemeinsam mit einer halbleitenden ersten Elektrode (20) als kombinierte Elektrode ausgeführt ist.

16. Elektronisches Bauelement (10) nach einem der Ansprüche 1 bis 15, **dadurch gekennzeichnet, dass** zwischen der zweiten Elektrode (16) und der molekularen Schicht (18) oder zwischen der Diode (22) und der molekularen Schicht (18) eine Zwischenschicht angeordnet ist, wobei die Zwischenschicht ausgewählt ist aus einem oxidischen oder fluoridischen Material.

17. Elektronisches Bauelement (10) nach einem der Ansprüche 1 bis 16, **dadurch gekennzeichnet, dass** das Bauelement (10) eine Vielzahl von Schaltelementen (1) aufweist, wobei die ersten Elektroden (20) und zweiten Elektroden (16) der Schaltelemente (1) eine Kreuzdrahtvorrichtung ausbilden.

18. Elektronisches Bauelement (10) nach einem der Ansprüche 1 bis 17, **dadurch gekennzeichnet, dass** die Schaltelemente eingerichtet sind, zwischen einem Zustand mit hohem elektrischen Widerstand und einem Zustand mit geringem elektrischen Widerstand zu wechseln, wobei der Quotient zwischen hohem elektrischen Widerstand und niedrigem elektrischen Widerstand zwischen 10 und 100000 liegt.

19. Elektronisches Bauelement nach einem der Ansprüche 1 bis 18, **dadurch gekennzeichnet, dass** es als memristives Bauelement ausgestaltet ist.

20. Verfahren zum Betrieb eines elektronischen Bauelements (10) nach einem der Ansprüche 1 bis 19, **dadurch gekennzeichnet, dass** ein Schaltelement (1) des elektronischen Bauelements (10) in einen Zustand hohen elektrischen Widerstands geschaltet wird, indem eine korrespondierende erste Elektrode (20) auf ein erstes elektrisches Potential und eine korrespondierende zweite Elektrode (16) auf ein zweites elektrisches Potential gelegt wird, wobei der Betrag der Spannung zwischen den beiden Elektroden (16, 20) größer ist, als eine erste Schaltspannung und das erste Potential größer als das zweite Potential ist, ein Schaltelement (1) des elektronischen Bauelements (10) in einen Zustand geringen elektrischen Widerstands geschaltet wird, indem eine korrespondierende erste Elektrode (20) auf ein drittes elektrisches Potential und eine korrespondierende zweite Elektrode (16) auf ein viertes elektrisches Potential gelegt wird, wobei der Betrag der Spannung zwischen den beiden Elektroden (16, 20) größer ist, als eine zweite Schaltspannung und das vierte Potential größer als das dritte Potential ist, und der Zustand eines Schaltelements bestimmt wird, indem zwischen korrespondierenden Elektroden (16, 20) eine Auslesespannung angelegt wird, deren Betrag kleiner als die erste und die zweite Schaltspannung ist und der fließende Strom gemessen wird.

21. Verwendung eines elektronischen Bauelements nach einem der Ansprüche 1 bis 8 als memristives elektronisches Bauelement.

## Claims

1. Electronic component (10) comprising a plurality of switching elements (1) which comprise, in this sequence,
a first electrode (20),
a molecular layer (18), bonded to a substrate, and
a second electrode (16),
where the first electrode (20) represents the substrate for the molecular layer,
**characterised in that** the molecular layer essentially consists of molecules (M) which contain a connecting group (V) and an end group (E) having a polar or ionic function and where the molecules (M) represent one or more compounds of the formula (I),
~ (A)ₛ-(V)-[Y¹-(Z¹-Y²)ₘ]ₜ-(E) (I)
in which
A denotes an anchor group;
V denotes a C₁-C₂₅-alkylene group, which may contain one or more functional groups and/or one or more 3- to 6-membered, saturated or partially unsaturated, alicyclic or heterocyclic rings in the chain and in which one or more H atoms may be replaced by halogen;
Y¹ and Y² in each case, independently of one another, denote an aromatic or alicyclic group, preferably having 4 to 25 C atoms, which may also contain condensed rings and which may be mono- or polysubstituted by a group R^{L};
R^{L} in each case independently denotes OH, SH, SR°, -(CH₂)ₙ-OH, F, Cl, Br, I, -CN, -NO₂, -NCO, -NCS, -OCN, -SCN, -C(=O)N(R°)₂, -C(=O)R°, -N(R°)₂, -(CH₂)ₙ-N(R°)₂, optionally substituted silyl, optionally substituted aryl or cycloalkyl having 6-20 C atoms or linear or branched alkyl, alkoxy, alkylcarbonyl, alkoxycarbonyl, alkylcarbonyloxy or alkoxycarbonyloxy having 1-25 C atoms, in which one or more H atoms may be replaced by F or Cl and in which two vicinal groups R^{L} together may optionally be =O;
n denotes 1, 2, 3 or 4;
Z¹ in each case independently denotes a single bond, -O-, -S-, -CO-, -CO-O-, -O-CO-, -O-CO-O-, -OCH₂, -CH₂O-, -SCH₂-, -CH₂S-, -CF₂O-, -OCF₂-, -CF₂S-, -SCF₂-, -(CH₂)ₙ-, -CF₂CH₂-, -CH₂CF₂₋, -(CF₂)ₙ-, -CH=CH-, -CF=CF-, -C=C-, -CH=CH-COO-, -OCO-CH=CH- or -CR°₂;
R° in each case, independently of one another, denotes H or C₁-C₁₂-alkyl;
m denotes 0, 1, 2, 3, 4 or 5;
s and t, independently of one another, denote 0 or 1,
and
~ indicates the bond to the substrate,
and where the end group (E) is a polar end group which has at least one bond in which the electronegativity difference between the atoms involved is at least 0.5, selected from CN, SCN, NO₂, (C₁-C₄)-haloalkyl, preferably CF₃, (C₁-C₄)-haloalkoxy, preferably OCF₃, -S-(C₁-C₄)-haloalkyl, preferably SCF₃, S(O)₂-(C₁-C₄)-haloalkyl, preferably SO₂CF₃, SFs, OSFs, N(C₁-C₄-haloalkyl)₂, preferably N(CF₃)₂, N(CN)₂ and (C₆-C₁₂)-haloaryl, preferably mono-, di- or trifluorophenyl,
or where the end group (E) is a redox-inactive cationic or anionic group selected respectively from imidazolium, pyridinium, pyrrolidinium, guanidinium, uronium, thiouronium, piperidinium, morpholinium, ammonium and phosphonium groups or halides, borates, sulfonates, carboxylates, phosphates, phosphinates, perfluorinated alkylsulfonates and -carboxylates, imide anions and amide anions, preferably optionally substituted tetraphenylborates, tetrafluoroborate, trifluoroacetate, triflate, hexafluorophosphate, phosphinates and optionally substituted tosylates, where the counterions of the cationic and anionic groups are selected respectively from the anionic and cationic groups mentioned.

2. Electronic component (10) according to Claim 1, where the connecting group (V) is a linear or branched C₁-C₂₅-alkylene group, in which one or more non-adjacent CH₂ groups may in each case be replaced by -C=C-, -CH=CH-, -NR'-, -O-, -S-, -CO-, -CO-O-, -O-CO-, -O-CO-O- or a 3-6-membered, saturated or partially unsaturated, alicyclic or heterocyclic ring, where N, O and/or S are not bonded directly to one another, in which one or more tertiary carbon atoms (CH groups) may be replaced by N and in which one or more hydrogen atoms may be replaced by halogen, where R' in each case, independently of one another, denotes H or C₁-C₁₂-alkyl.

3. Electronic component (10) according to Claim 2, where the connecting group (V) is a linear or branched C₁-C₁₀-alkylene group, in which one or more non-adjacent CH₂ groups may in each case be replaced by -O-, -S-, or a 3-6-membered, saturated alicyclic ring, where O and/or S are not bonded directly to one another, and in which one or more hydrogen atoms may be replaced by F and/or Cl.

4. Electronic component (10) according to one of Claims 1 to 3, where the molecules (M), besides the connecting group (V) and the end group (E), contain an anchor group (A), via which the connecting group is bonded to the substrate.

5. Electronic component (10) according to Claim 4, where the anchor group (A) is bonded to the substrate via a covalent bond.

6. Electronic component (10) according to Claim 5, where the anchor group (A) is bonded to the substrate by physisorption.

7. Electronic component (10) according to Claim 5 or 6, where the anchor group (A) is selected from carboxylate, phosphonate, alcoholate, arylate, preferably phenolate, thiolate and sulfonate groups or fullerene derivatives, preferably [60]PCBM (methyl [6,6]-phenyl-C61-butanoate) and [70]PCBM (methyl [6,6]-phenyl-C71-butanoate).

8. Electronic component (10) according to one of Claims 1 to 7, **characterised in that** the molecular layer is a molecular monolayer.

9. Electronic component (10) according to one of Claims 1 to 8, **characterised in that** the first electrode (20) consists of a material selected from element semiconductors, preferably Si, Ge, C (diamond, graphite, graphene), Sn (alpha modification) and Se, compound semiconductors from groups III-V, preferably GaAs, InAs, InP, GaSb and GaN, and compound semiconductors from groups II-VI, preferably CdSe and ZnS, in various doping, metals, preferably Au, Ag, Cu, Al and Mg, and conductive, oxidic materials, preferably ITO, IGO, IGZO, AZO and FTO.

10. Electronic component (10) according to one of Claims 1 to 9, **characterised in that** the molecular layer is bonded to an oxidic or fluoridie interlayer.

11. Electronic component (10) according to Claim 10, **characterised in that** the oxidic or fluoridic interlayer is formed from TiO₂, Al₂O₃, HfO₂, SiO₂ or LiF.

12. Electronic component (10) according to one of Claims 1 to 11, **characterised in that** the second electrode (16) consists of a conducting or semiconducting material or a combination of a plurality of these materials selected from Hg, In, Ga, InGa, Ag, Au, Cr, Pt, PdAu, Pb, Al, Mg, CNT, graphene and conductive polymers.

13. Electronic component (10) according to one of Claims 1 to 12, **characterised in that** the first electrode (20) and the second electrode (16) are metallic conductors or **in that** the second electrode (16) is a metallic conductor and the first electrode (20) is semiconducting.

14. Electronic component (10) according to one of Claims 1 to 13, **characterised in that** a diode (22) is arranged between the first electrode (20) and the molecular layer (18) or between the second electrode (16) and the molecular layer (18).

15. Electronic component (10) according to Claim 14, **characterised in that** the diode (22) comprises an n+-doped layer (24) and a p+-doped layer (26), where the n+-doped layer (24) or the p+-doped layer (26) is implemented as a combined electrode together with a semiconducting first electrode (20).

16. Electronic component (10) according to one of Claims 1 to 15, **characterised in that** an interlayer is arranged between the second electrode (16) and the molecular layer (18) or between the diode (22) and the molecular layer (18), where the interlayer is selected from an oxidic or fluoridic material.

17. Electronic component (10) according to one of Claims 1 to 16, **characterised in that** the component (10) has a multiplicity of switching elements (1), where the first electrodes (20) and second electrodes (16) of the switching elements (1) form a crossbar array.

18. Electronic component (10) according to one of Claims 1 to 17, **characterised in that** the switching elements are set up to change between a state of high electrical resistance and a state of low electrical resistance, where the quotient between high electrical resistance and low electrical resistance is between 10 and 100,000.

19. Electronic component according to one of Claims 1 to 18, **characterised in that** it is designed as a memristive component.

20. Method for operating an electronic component (10) according to one of Claims 1 to 19, **characterised in that** a switching element (1) of the electronic component (10) is switched into a state of high electrical resistance by setting a corresponding first electrode (20) to a first electrical potential and setting a corresponding second electrode (16) to a second electrical potential, where the value of the voltage between the two electrodes (16, 20) is greater than a first switching voltage and the first potential is greater than the second potential, a switching element (1) of the electronic component (10) is switched into a state of low electrical resistance by setting a corresponding first electrode (20) to a third electrical potential and setting a corresponding second electrode (16) to a fourth electrical potential, where the value of the voltage between the two electrodes (16, 20) is greater than a second switching voltage and the fourth potential is greater than the third potential, and the state of a switching element is determined by applying a reading voltage whose value is smaller than the first and second switching voltages between corresponding electrodes (16, 20) and measuring the current flowing.

21. Use of an electronic component according to one of Claims 1 to 8 as memristive electronic component.

## Revendications

1. Composant électronique (10) comprenant une pluralité d'éléments de commutation (1) qui comprennent, dans cette séquence,
une première électrode (20),
une couche moléculaire (18), liée à un substrat, et
une deuxième électrode (16),
où la première électrode (20) représente le substrat pour la couche moléculaire,
**caractérisé en ce que** la couche moléculaire est constituée essentiellement de molécules (M) qui contiennent un groupement de connexion (V) et un groupement terminal (E) ayant une fonction polaire ou ionique et où les molécules (M) représentent un ou plusieurs composés de formule (I),
~ (A)ₛ-(V)-[Y¹-(Z¹-Y²)ₘ]ₜ-(E) (I)
dans laquelle
A désigne un groupement d'ancrage ;
V désigne un groupement C₁-C₂₅-alkylène, qui peut contenir un ou plusieurs groupements fonctionnels et/ou un ou plusieurs cycles alicycliques ou hétérocycliques de 3 à 6 chaînons, saturés ou partiellement insaturés, dans la chaîne et où un ou plusieurs atomes de H peuvent être remplacés par halogène ;
Y¹ et Y² dans chaque cas, indépendamment l'un de l'autre, désignent un groupement aromatique ou alicyclique, préférablement ayant de 4 à 25 atomes de C, qui peut également contenir des cycles condensés et qui peut être mono- ou polysubstitué par un groupement R^{L} ;
R^{L} dans chaque cas indépendamment désigne OH, SH, SR°, -(CH₂)ₙ-OH, F, Cl, Br, I, -CN, -NO₂, -NCO, -NCS, -OCN, -SCN, -C(=O)N(R°)₂, -C(=O)R°, -N(R°)₂, -(CH₂)ₙ-N(R°)₂, silyle éventuellement substitué, aryle ou cycloalkyle éventuellement substitué ayant 6-20 atomes de C ou alkyle, alcoxy, alkylcarbonyle, alcoxycarbonyle, alkylcarbonyloxy ou alcoxycarbonyloxy linéaire ou ramifié ayant 1-25 atomes de C, où un ou plusieurs atomes de H peuvent être remplacés par F ou Cl et où deux groupements vicinaux R^{L} peuvent éventuellement être ensemble =O ;
n désigne 1, 2, 3 ou 4 ;
Z¹ dans chaque cas indépendamment, désigne une liaison simple, -O-, -S-, -CO-, -CO-O-, -O-CO-, -O-CO-O-, -OCH₂, -CH₂O-, -SCH₂-, -CH₂S-, -CF₂O-, -OCF₂-, -CF₂S-, -SCF₂-, -(CH₂)ₙ-, -CF₂CH₂-, -CH₂CF₂₋, -(CF₂)ₙ-, -CH=CH-, -CF=CF-, -C=C-, -CH=CH-COO-, -OCO-CH=CH- ou -CR°₂ ;
R° dans chaque cas, indépendamment les uns des autres, désigne H ou C₁-C₁₂-alkyle ;
m désigne 0, 1, 2, 3, 4 ou 5 ;
s et t, indépendamment l'un de l'autre, désignent 0 ou 1,
et
~ indique la liaison au substrat,
et où le groupement terminal (E) est un groupement terminal polaire qui possède au moins une liaison dans laquelle la différence d'électronégativité entre les atomes impliqués est d'au moins 0,5, choisi parmi CN, SCN, NO₂, (C₁-C₄)halogénoalkyle, préférablement CF₃, (C₁-C₄)halogénoalcoxy, préférablement OCF₃, -S-(C₁-C₄)-halogénoalkyle, préférablement SCF₃, S(O)₂-(C₁-C₄)halogénoalkyle, préférablement SO₂CF₃, SFs, OSFs, N(C₁-C₄-halogénoalkyl)₂, préférablement N(CF₃)₂, N(CN)₂ et (C₆-C₁₂)halogénoaryle, préférablement mono-, di- ou trifluorophényle,
ou où le groupement terminal (E) est un groupe cationique ou anionique inactif sur le plan de l'oxydoréduction choisi respectivement parmi les groupements imidazolium, pyridinium, pyrrolidinium, guanidinium, uronium, thiouronium, pipéridinium, morpholinium, ammonium et phosphonium ou les halogénures, borates, sulfonates, carboxylates, phosphates, phosphinates, alkylsulfonates et -carboxylates perfluorés, les anions imide et les anions amide, préférablement les tétraphénylborates éventuellement substitués, tétrafluoroborate, trifluoroacétate, triflate, hexafluorophosphate, phosphinates, et tosylates éventuellement substitués, où les contreions des groupements cationiques et anioniques sont choisis respectivement parmi les groupements anioniques et cationiques mentionnés.

2. Composant électronique (10) selon la revendication 1, dans lequel le groupement de connexion (V) est un groupement C₁-C₂₅-alkylène linéaire ou ramifié, où un ou plusieurs groupements CH₂ non adjacents peuvent dans chaque cas être remplacés par -C=C-, -CH=CH-, -NR'-, -O-, -S-, -CO-, -CO-O-, -O-CO-, -O-CO-O- ou un cycle alicyclique ou hétérocyclique de 3-6 chaînons, saturé ou partiellement insaturé, où N, O et/ou S ne sont pas liés directement les uns aux autres, où un ou plusieurs atomes de carbone tertiaire (groupements CH) peuvent être remplacés par N et où un ou plusieurs atomes d'hydrogène peuvent être remplacés par halogène, où R' dans chaque cas, indépendamment les uns des autres, désigne H ou C₁-C₁₂-alkyle.

3. Composant électronique (10) selon la revendication 2, dans lequel le groupement de connexion (V) est un groupement C₁-C₁₀-alkylène linéaire ou ramifié, où un ou plusieurs groupements CH₂ non adjacents peuvent dans chaque cas être remplacés par -O-, -S-, ou un cycle alicyclique saturé de 3-6 chaînons, où O et/ou S ne sont pas liés directement les uns aux autres, et où un ou plusieurs atomes d'hydrogène peuvent être remplacés par F et/ou Cl.

4. Composant électronique (10) selon l'une parmi les revendications 1 à 3, dans lequel les molécules (M), outre le groupement de connexion (V) et le groupement terminal (E), contiennent un groupement d'ancrage (A), via lequel le groupement de connexion est lié au substrat.

5. Composant électronique (10) selon la revendication 4, dans lequel le groupement d'ancrage (A) est lié au substrat via une liaison covalente.

6. Composant électronique (10) selon la revendication 5, dans lequel le groupement d'ancrage (A) est lié au substrat par physisorption.

7. Composant électronique (10) selon la revendication 5 ou 6, dans lequel le groupement d'ancrage (A) est choisi parmi les groupements carboxylate, phosphonate, alcoolate, arylate, préférablement phénolate, thiolate et sulfonate ou les dérivés de fullerène, préférablement [60]PCBM ( [6,6]-phényl-C61-butanoate de méthyle) et [70]PCBM ( [6,6]-phényl-C71-butanoate de méthyle).

8. Composant électronique (10) selon l'une parmi les revendications 1 à 7, **caractérisé en ce que** la couche moléculaire est une monocouche moléculaire.

9. Composant électronique (10) selon l'une parmi les revendications 1 à 8, **caractérisé en ce que** la première électrode (20) est constituée d'un matériau choisi parmi les semi-conducteurs élémentaires, de préférence Si, Ge, C (diamant, graphite, graphène), Sn (modification alpha) et Se, les semi-conducteurs composés des groupes III-V, de préférence GaAs, InAs, InP, GaSb et GaN, et les semi-conducteurs composés des groupes II-VI, de préférence CdSe et ZnS, dans différents métaux de dopages, de préférence Au, Ag, Cu, Al et Mg, et des matériaux oxygénés conducteurs, de préférence ITO, IGO, IGZO, AZO et FTO.

10. Composant électronique (10) selon l'une parmi les revendications 1 à 9, **caractérisé en ce que** la couche moléculaire est liée à une couche intermédiaire oxygénée ou fluorée.

11. Composant électronique (10) selon la revendication 10, **caractérisé en ce que** la couche intermédiaire oxygénée ou fluorée est formée par TiO₂, Al₂O₃, HfO₂, SiO₂ ou LiF.

12. Composant électronique (10) selon l'une parmi les revendications 1 à 11, **caractérisé en ce que** la deuxième électrode (16) est constituée d'un matériau conducteur ou semi-conducteur ou d'une combinaison d'une pluralité de ces matériaux choisis parmi Hg, In, Ga, InGa, Ag, Au, Cr, Pt, PdAu, Pb, Al, Mg, CNT, le graphène et les polymères conducteurs.

13. Composant électronique (10) selon l'une parmi les revendications 1 à 12, **caractérisé en ce que** la première électrode (20) et la deuxième électrode (16) sont des conducteurs métalliques ou **en ce que** la deuxième électrode (16) est un conducteur métallique et la première électrode (20) est semi-conductrice.

14. Composant électronique (10) selon l'une parmi les revendications 1 à 13, **caractérisé en ce qu'**une diode (22) est disposée entre la première électrode (20) et la couche moléculaire (18) ou entre la deuxième électrode (16) et la couche moléculaire (18).

15. Composant électronique (10) selon la revendication 14, **caractérisé en ce que** la diode (22) comprend une couche dopée n+ (24) et une couche dopée p+ (26), où la couche dopée n+ (24) ou la couche dopée p+ (26) est mise en oeuvre en tant qu'électrode combinée avec une première électrode semi-conductrice (20).

16. Composant électronique (10) selon l'une parmi les revendications 1 à 15, **caractérisé en ce qu'**une couche intermédiaire est disposée entre la deuxième électrode (16) et la couche moléculaire (18) ou entre la diode (22) et la couche moléculaire (18), où la couche intermédiaire est choisie parmi un matériau oxygéné ou fluoré.

17. Composant électronique (10) selon l'une parmi les revendications 1 à 16, **caractérisé en ce que** le composant (10) comporte une pluralité d'éléments de commutation (1), où les premières électrodes (20) et les deuxièmes électrodes (16) des éléments de commutation (1) forment un réseau de barres croisées.

18. Composant électronique (10) selon l'une parmi les revendications 1 à 17, **caractérisé en ce que** les éléments de commutation sont conçus pour passer d'un état de résistance électrique élevée à un état de résistance électrique faible, où le quotient entre la résistance électrique élevée et la résistance électrique faible est compris entre 10 et 100 000.

19. Composant électronique selon l'une parmi les revendications 1 à 18, **caractérisé en ce qu'**il est conçu comme composant memristor.

20. Méthode destinée au fonctionnement d'un composant électronique (10) selon l'une parmi les revendications 1 à 19, **caractérisée en ce qu'**un élément de commutation (1) du composant électronique (10) est commuté dans un état de résistance électrique élevée en réglant une première électrode (20) correspondante à un premier potentiel électrique et en réglant une deuxième électrode (16) correspondante à un deuxième potentiel électrique, la valeur de la tension entre les deux électrodes (16, 20) étant supérieure à une première tension de commutation et le premier potentiel étant supérieur au deuxième potentiel, un élément de commutation (1) du composant électronique (10) est commuté dans un état de faible résistance électrique en réglant une première électrode (20) correspondante à un troisième potentiel électrique et en réglant une deuxième électrode (16) correspondante à un quatrième potentiel électrique, où la valeur de la tension entre les deux électrodes (16, 20) est supérieure à une deuxième tension de commutation et le quatrième potentiel est supérieur au troisième potentiel, et l'état d'un élément de commutation est déterminé en appliquant une tension de lecture dont la valeur est inférieure à la première et deuxième tension de commutation entre les électrodes (16, 20) correspondantes et en mesurant le courant qui circule.

21. Utilisation d'un composant électronique selon l'une parmi les revendications 1 à 8, comme composant électronique memristor.
